# EUROPÄISCHE PATENTSCHRIFT

(11) **EP 2 748 107 B1**
(45) Veröffentlichungstag und Bekanntmachung des Hinweises auf die Patenterteilung: **15.08.2018**
(21) Anmeldenummer: 12750327.4
(22) Anmeldetag: 20.08.2012
(51) Int. Cl.: B81C 1/00, B81B 7/00, C30B 11/12, C30B 29/06, C30B 29/60, G01N 33/487, B82Y 10/00

(54) **VERFAHREN ZUR HERSTELLUNG UND AUSRICHTUNG VON NANOWIRES UND ANWENDUNGEN EINES SOLCHEN VERFAHRENS**
METHOD FOR PRODUCING AND ALIGNING NANOWIRES AND APPLICATIONS OF SUCH A METHOD
PROCÉDÉ DE FABRICATION ET D'ORIENTATION DE NANOFILS ET APPLICATIONS D'UN TEL PROCÉDÉ

(30) Priorität: 22.08.2011 EP 11006842
(43) Veröffentlichungstag der Anmeldung: 02.07.2014
(73) Patentinhaber: Albuschies, Jörg, 8006 Zürich (CH)
(72) Erfinder: Albuschies, Jörg, 8006 Zürich (CH)
(74) Vertreter: Bremi, Tobias Hans
(86) Internationale Anmeldenummer: PCT/EP2012/003530
(87) Internationale Veröffentlichungsnummer: WO 2013/026561

(56) Entgegenhaltungen:
- WO-A2-2007/136755
- US-A1- 2005 181 587
- US-A1- 2008 224 122
- ZHIYONG FAN ET AL: "Wafer-Scale Assembly of Highly Ordered Semiconductor Nanowire Arrays by Contact Printing", NANO LETTERS, Bd. 8, Nr. 1, 1. Januar 2008 (2008-01-01), Seiten 20-25, XP055017384, ISSN: 1530-6984, DOI: 10.1021/nl071626r
- YI-KUEI CHANG ET AL: "The fabrication of ZnO nanowire field-effect transistors by roll-transfer printing", NANOTECHNOLOGY, IOP, BRISTOL, GB, Bd. 20, Nr. 19, 13. Mai 2009 (2009-05-13), Seite 195302, XP020152941, ISSN: 0957-4484, DOI: 10.1088/0957-4484/20/19/195302
- CHANG YING-LAN ET AL: "Controlled formation of individually seeded, electrically addressable silicon nanowire arrays for device integration", APPLIED PHYSICS LETTERS, A I P PUBLISHING LLC, US, vol. 89, no. 22, 1 December 2006 (2006-12-01), pages 223123-223123, XP012087351, ISSN: 0003-6951, DOI: 10.1063/1.2398900

## Beschreibung

### TECHNISCHES GEBIET

Die vorliegende Erfindung betrifft Verfahren zur Herstellung und Ausrichtung von Nanowires und Anwendungen eines solchen Verfahrens, zudem betrifft die vorliegende Erfindung Verfahren zur Herstellung von Nano-Trichter in einem Einkristall-Siliziumsubstrat, sowie insbesondere Verwendungen von diesen Verfahren respektive den daraus resultierenden Strukturen zur Herstellung eines Molekül-Sensors mit hoher Empfindlichkeit und Verfahren zu dessen Betrieb.

### STAND DER TECHNIK

Für die elektronische Kontaktierung von Nanodrähten (Nanowires) auf einem isolierenden Substrat in lateraler Weise ist eine Ausrichtung der Nanodrähte parallel zum Substrat notwendig. Die Schwierigkeit liegt darin, Nanodrähte parallel auf das isolierende Substrat zu bekommen. Für diesen Schritt werden derzeit zwei Ansatze verfolgt.
a) gerichtetes laterales Wachstum, so dass keine nachträgliche Ausrichtung notwendig ist. Dies ist sehr schwierig zu realisieren, da isolierende Substrate amorph sind und Nanodrähte keine kristalline Wachstumsrichtung vorgeben können.
b) Die Nanodrähte können von einem Wachstumssubstrat abgelöst werden, und (meist in Flüssigkeit suspendiert) auf ein anderes Substrat transferiert und dort lateral auf dem isolierenden Substrat deponiert werden (elektrophorese/microfluidics/contact printing ...). Problem: Lokalisierung und Orientierung der Nanodrähte ist schwierig zu realisieren, besonders im Hinblick auf industrielle parallele Fertigung mehrerer Bauelemente auf Wafer-Ebene. Besonders im Bezug auf die Kontaktierung einzelner/weniger Nanodrähte zwischen definierten Metallelektroden gibt es derzeit keine wirtschaftlich parallelisierbare Methode.

Aus Ying-Lan Chang et al. (Appl Phys. Lett. 89, 223123 (2006)) ist die kontrollierte Herstellung eines geraden Nanowires bekannt, der anschliessend zwischen zwei Kontakten angeordnet werden kann. Hierzu werden auf der Oberfläche eines isolierenden Substrats katalytisch aktive Gold-Nanopartikel mit einem Durchmesser im Bereich von ca. 40nm auf dem Substrat abgelegt und bei einer Temperatur im Bereich von 450-550 °C während ungefähr 10 Minuten die Oberfläche und die darauf abgelagerten Nanopartikel einem Gasstrom enthaltend Si2H6 ausgesetzt, wobei sich vom Substrat abstehende Nanowires einer Länge im Bereich 1-5 µm bilden. Diese Nanowires werden mit einer Ionenstrahlbehandlung auf dem Substrat abgelegt worauf wenigstens ein Nanowire an zwei unterschiedlichen Stellen mit Elektronenstrahllithographie kontaktiert wird.

### DARSTELLUNG DER ERFINDUNG

Mittels (grober) optischer Lithographie im Mikrometerbereich (> 1 µm) und einer sehr groben lateralen Ausrichtungstoleranz für optische Lithographie (> 1 µm) lassen sich einzelne Mikrometer lange Nanodrähte lateral auf einem isolierenden Substrat elektronisch kontaktieren. Die Nanodrähte werden für diesen Prozess auf Mikrometer grossen lokal definierten runden Nukleationsflächen auf dem finalen Substrat erzeugt (gewachsen), ohne dass sie für den Prozess von einem Substrat auf ein anderes transferiert werden müssen. Dabei haben die Nanodrähte eine willkürliche Orientierung im Bezug zur Substratoberfläche (3D). Ein neuartiger Ausrichtungsprozess erlaubt es, die willkürliche Orientierung der (an einem Ende mit dem Substrat verankerten) Nanodrähte parallel zum Substrat auszurichten (2D), während sie noch mit einer Seite auf dem Substrat verankert sind. Die Nanodrähte können radial von ihrem Wachstumsort nach aussen hin auf das Substrat ausgerichtet werden. Dies ermöglicht die elektrische Kontaktierung einzelner - lateral auf dem Substrat liegender - Nanodrähte mit leitenden Metallelektroden mittels optischer Lithographie. Die Nanodrähte werden mit einer kreisförmigen Elektrode über dem Zentrum der Nukleationsfläche kontaktiert. Die zweite Elektrode liegt in ringförmiger Anordnung ausserhalb der ersten Elektrode und kontaktiert die Enden der nach aussen gerichteten Nanodrähte. Eine Gate-Elektrode kann für Transistoranwendungen ebenfalls zwischen die beiden kreis- und ringförmigen Elektroden über die Nanodrähte aufgebracht werden, um Nanowire-Transistoren herzustellen.

Das hier vorgestellte Verfahren zur Herstellung und Ausrichtung von Nanowires kommt mit zwei Mikrometer ungenauen optischen Lithographieschritten auf demselben Substrat aus, um einen vollständig funktionsfähigen elektronischen Nanowire-Biosensor herzustellen.
a) Lithographie 1: Lokalisierung der Gold Katalysatoren auf bevorzugtermassen kreisrunden Mikrometer grossen Flächen
b) Wachstum der Nanodrahte 20-40 nm Durchmesser, 10-20 Mikrometer Lange
c) Ausrichtung der willkürlich 3D orientierten Nanodrahte parallel zum Substrat. Dabei liegen die Nanodrähte typischerweise radial nach aussen, wie die Speichen eines Rades, ausgehend von der Radnabe.
d) Lithographie 2: Gleichzeitige Deposition von bevorzugtermassen kreisförmigen Elektroden im Zentrum auf der "Radnabe" und aussen als Felge über den Enden der Nanodrähte. Die äussere Elektrode muss eine Öffnung aufweisen, wodurch die innere Elektrode mit einer Leiterbahn kontaktiert werden kann.

Die Anzahl der kontaktierten Nanodrahte kann auf zwei Arten kontrolliert werden.
a) Dichte der Katalysatorpartikel auf dem ursprünglichen Nukleationsareal definiert die totale Anzahl der Nanodrähte
b) Die Länge der Nanodrahte weist eine Gauss-Normalverteilung auf. Die Entfernung der Aussenelektrode zum Zentrum kann demnach auf einen Teil Längenverteilung abgestimmt werden. Die übrigen Nanodrähte liegen passiv/redundant und nicht kontaktiert auf dem Substrat

Grösster industrieller/wirtschaftlicher Vorteil:
- Kostengünstige Herstellung.
- Keine aufwendigen Alignment-Techniken notwendig, keine teure Elektronenstrahllithographie für die Kontaktierung von Nanostrukturen.
- Parallelisierbar für den Einsatz auf Waferscale. Vergleichbare Bauelemente konnten bisher nur in aufwendigen einzelnen Labor-Prototypen realisiert werden. Oder über nanoscale optische Lithographie, die extrem teure und aufwändige Anlagen bedarf.

Zur industriellen parallelen Fertigung kompletter Bauelemente nach dieser Methode sind nötig: eine 25 Jahre alte Lithographie-Anlage; ein LPCVD Ofen; eine Metallisierungsanlage.

Spezifisch betrifft die vorliegende Erfindung ein Verfahren nach Anspruch 1 zur Herstellung einer Leiterstruktur mit wenigstens einem Nanowire, insbesondere einem Silizium-Nanowire, mit einem Durchmesser von weniger als 50 nm, der über wenigstens zwei Stellen über Elektroden kontaktiert ist, und wobei der wenigstens eine Nanowire und die Elektroden in einer Ebene auf einem Substrat angeordnet sind. Das Verfahren ist dabei durch die Merkmale von Anspruch 1 gekennzeichnet, insbesondere dadurch, dass folgende Verfahrensschritte durchgeführt werden:
a) dass auf der Oberfläche eines isolierenden Substrats katalytisch aktive Metall-Nanopartikel mit einem Durchmesser im Bereich von 0.5-50 nm auf lokal definierten Nukleationsflächen abgelegt werden,
b) dass bei einer Temperatur im Bereich von 300-1100 °C während einer Zeitdauer im Bereich von 10-200 min die Oberfläche und die darauf abgelagerten Metall-Nanopartikel einem Gasstrom enthaltend wenigstens eine gasförmige Siliziumkomponente ausgesetzt werden, wobei sich wenigstens ein vom Substrat abstehender Nanowire einer Länge im Bereich von 5-200 µm bildet (typischerweise werden Längen im Bereich von 10-100 µm, bevorzugt im Bereich von 20-50 µm, ausgebildet);
c) dass dieser wenigstens eine von der Oberfläche des Substrats abstehende Nanowire durch Auflegen eines Sekundärsubstrats mit einer der Oberfläche des isolierenden Substrats korrespondierenden Kontaktfläche in eine Ebene abgelegt wird;
d) dass der auf dem isolierenden Substrat abgelegte wenigstens eine Nanowire an zwei unterschiedlichen Stellen mit Elektroden kontaktiert wird, wozu eine erste Elektrode über dem Zentrum der entsprechenden Nukleationsfläche und eine zweite Elektrode in ringförmiger Anordnung um die erste Elektrode erzeugt werden.
Der angegebene Temperaturbereich von 300-1100 °C für die Prozessführung im Rahmen des Schrittes b) gilt insbesondere dann, wenn als KatalysatorPartikel (Metall-Nanopartikel) Goldpartikel eingesetzt werden. Werden andere Materialien wie beispielsweise Aluminium-Partikel oder Titandioxid-Partikel eingesetzt, so können gegebenenfalls auch höhere Temperaturen bis 1500 °C oder sogar 2000 °C eingesetzt Dieser Temperaturbereich ist jedoch nicht Teil der Erfindung. Die Wachstumstemperatur für verschiedenartige Nanodrähte erstreckt sich im Prinzip von Raumtemperatur bis zu einer Obergrenze, die auch deutlich über 1000° liegen kann. Es wurde Silizium-Nanodraht-Wachstum bei 1200°C berichtet. Für Silizium liegt die höchste mögliche Temperatur nahe am Schmelzpunkt, der der sich bei 1410°C befindet.

Für andere Materialien wird die Obergrenze der Temperatur beim Wachstumsprozess ähnlich nahe am jeweiligen Schmelzpunkt liegen. Die Prozess-Parameter-Fenster sind entsprechend ggf. gross. Der Prozess funktioniert im Prinzip mit so kurzen Nanowires, wie die optische Lithographie in der Lage ist, eine genaue Ausrichtung zwischen den beiden Lithographieschritten für a) Katalysatordeposition und b) Elektrodendeposition zu gewährleisten. D.h. die minimale Länge der Nanowires ist durch die maximale Ausrichtungsungenauigkeit/Toleranz zwischen den beiden Lithographieschritten beschränkt. Bei modernen Geräten kann sich die Aurichtungstoleranz der Lithographieschritte in einem Bereich von nur wenigen hundert Nanometern, ggf. sogar unter 100 nm befinden. Der wirtschaftliche Vorteil liegt jedoch daran, dass über längere Nanowires eine geringe Ausrichtungstoleranz von sehr günstigen (und älteren) Geräten bereits eine zuverlässig Kontaktierung der Nanodrähte über Metallelektroden ermöglicht.

Zum Schritt c) sei folgendes ergänzend bemerkt: es kann sich bei der Kontaktebene des Sekundärsubstrats um eine flache Ebene oder aber auch um eine gekrümmte Ebene handeln. Bei einer gekrümmten Ebene als Kontaktebene kann das Sekundärsubstrat beispielsweise auf der Oberfläche des Primärsubstrats abrollen und so die Nanowires in Flachlage bringen. Handelt es sich um eine korrespondierende flächig auf die Oberfläche gepresste Kontaktebene, ist dabei insbesondere wesentlich, dass diese Kontaktfläche des Sekundärsubstrats korrespondierend, d.h. im wesentlichen komplementär, zur an dieser Stelle ausgebildeten Oberfläche des Primärsubstrats ausgebildet ist, so dass die Strukturen auf die Ebene des Primärsubstrats gelegt werden.

Zudem eignet sich der vorgeschlagene Ausrichteprozess für die Nanodrähte erstaunlicherweise im Prinzip für jede eindimensionale Nanostruktur (Nanowires aus allen erhältlichen Materialien und ebenso Kohlenstoffnanoröhrchen). Im erfindungsgemäßen Verfahren werden jedoch ausschließlich Nanowires auf dem Substrat abgelegt. Um die Nanodrähte lateral und radial ausgerichtet auf ein Substrat zu bekommen, kann der Prozess, in dem der auf dem isolierenden Substrat abgelegte wenigstens eine Nanowire an zwei unterschiedlichen Stellen mit Elektroden kontaktiert wird, modifiziert werden.

Wie vorher beschrieben, werden die Nanodrähte durch ein Hilfsmittel auf dem Wachstumssubstrat "plattgedrückt". Überraschend ist dabei grundsätzlich, dass dies unerwarteterweise überhaupt möglich ist, der Fachmann würde davon ausgehen, dass bei einem solchen Prozess die Nanowire zerstört oder zumindest unbrauchbar gemacht würden. Eine eventuelle Anti-Haft Beschichtung des Hilfsmittels kann dabei das Anhaften der Nanodrähte am Hilfsmittel verhindern.

Gemäß einer ersten bevorzugten Ausführungsform ist das vorgeschlagene Verfahren dadurch gekennzeichnet, dass es sich beim isolierenden Substrat um ein Substrat aus Silizium, Siliziumdioxid oder Glas handelt. Mögliche weitere isolierende Substrate, die Siliziumoxid oder Glass mit gleicher Funktionalität ersetzen können sind Siliziumnitrid etc. Es ist an sich jedes elektrisch isolierende Substrat denkbar, das die jeweiligen Prozesstemperaturen aushält.

Eine weitere bevorzugte Ausführungsform ist dadurch gekennzeichnet, dass es sich bei den Metall-Nanopartikeln um Gold-Nanopartikel handelt, vorzugsweise mit einem Durchmesser im Bereich von 5-50 nm, vorzugsweise im Bereich von 20-45 nm. Erfindungsgemäß werden im Rahmen des Schrittes a) die Metall-Nanopartikel auf der Oberfläche des Substrats räumlich in gezielt begrenzten Bereichen abgelegt, typischerweise unter Verwendung von lithographischen Methoden, beispielsweise, indem eine Schicht aus einem Fotolack, insbesondere unter Verwendung von optischer Lithographie, aufgebracht wird, wobei, bevorzugt über selektive Belichtung über eine strukturierte Chrommaske, Löcher mit einem Durchmesser im Bereich von 0.02-10 µm, vorzugsweise 0.5-5 µm, in dieser Schicht aus Fotolack erzeugt werden (die Löcher erstrecken sich bis auf das Substrat), und indem
- entweder anschließend eine bevorzugt wässrige Lösung, welche Metall-Nanopartikel als Kolloide trägt, vorzugsweise Nanopartikel mit einem Durchmesser im Bereich von 0.5-500 nm, insbesondere vorzugsweise mit einem Durchmesser im Bereich von 5-150 nm, auf den Fotolack aufgebracht wird, wobei die Lösung abgedampft wird und wobei anschließend der Lack mit einem geeigneten Lösungsmittel, bevorzugt Aceton, entfernt wird,
- oder indem unter Verwendung von Elektronenstrahl-Metallverdampfung im Vakuum ein Metallfilm, insbesondere ein Goldfilm, auf diese Schicht aus Fotolack aufgebracht wird, vorzugsweise mit einer Schichtdicke im Bereich von 0.1-2 nm, und indem anschließend der Fotolack und das darauf befindliche Metall mittels eines geeigneten Lösungsmittels, bevorzugt Aceton, entfernt wird.

Eine weitere bevorzugte Ausführungsform des Verfahrens ist dadurch gekennzeichnet, dass im Schritt b) bei einer Temperatur im Bereich von 350-500 °C, vorzugsweise im Bereich von 400-480 °C, insbesondere vorzugsweise im Bereich von 450-470 °C gearbeitet wird.

Noch eine weitere bevorzugte Ausführungsform ist dadurch gekennzeichnet, dass als gasförmige Siliziumkomponente ein Silan oder ein Disilan, vorzugsweise in Kombination mit einem Trägergas, insbesondere bevorzugt Stickstoff oder Wasserstoff, eingesetzt wird.

Es kann bevorzugtermassen ein Gasfluss im Bereich von 50-200 sscm von gasförmiger Siliziumkomponente, insbesondere vom Silan respektive Disilan eingesetzt werden und ein Gasfluss im Bereich von 100-300 sscm Trägergas.

Generell erweist es sich als vorteilhaft, im Rahmen des Schrittes b) bei einem Gesamtdruck im Bereich von 1-50 mbar zu arbeiten, vorzugsweise im Bereich von 2-10 mbar über dem Substrat gehalten wird.

Eine weitere bevorzugte Ausführungsform des vorgeschlagenen Verfahrens ist dadurch gekennzeichnet, dass im Schritt a) die Nanopartikel in wenigstens einem, vorzugsweise in mehreren, voneinander getrennten, Nukleationsarealen abgelegt werden, so dass sich im Schritt b) eine Mehrzahl von Nanowires über jedem Nukleationsareal ausbildet, und dass bevorzugtermassen im Rahmen des Schrittes d) eine erste zentrale Elektrode über dem Nukleationsareal ein erstes Ende der Nanowires kontaktierend, vorzugsweise über eine Metallbedampfung oder eine fotolithographische Deposition, erzeugt wird, und eine zweite, wenigstens teilweise die erste Elektrode umlaufend ausgebildete zweite Elektrode, vorzugsweise über eine Metallbedampfung oder eine fotolithographische Deposition, erzeugt wird.

Ein Verfahren zur trockenchemischen Erzeugung einer Ausnehmung in einem kristallinen Substrat wird weiter beschrieben. Dieses trockenchemische Ätzverfahren ermöglicht es, kristalline Substrate, insbesondere monokristalline Substrate, ganz besonders monokristallines Silizium zu strukturieren.

Ein lokal platzierter Katalysator, insbesondere ein Goldkatalysator, ermöglicht die selektive Ätzung von Silizium in Abhängigkeit von der Kristallorientierung. Der Goldkatalysator vermindert die Bindungsenergie zwischen Siliziumatomen, die sich in der <100> Kristallebene befinden. Die Silizium <111> Oberfläche wird wesentlich langsamer geätzt, als die Silizium <100> Oberfläche. Das erlaubt es, lokal nanometergrosse Vertiefungen in eine Silizium <100> Oberfläche zu ätzen, deren Seitenwände die Silizium <111> Oberfläche haben. Dadurch entstehen pyramidenförmige Vertiefungen von geometrisch genau definierten Proportionen.

Die Vertiefungen haben exakt quadratisch- bis rechteckförmige Grundrisse, mit rechten Winkeln zwischen den Seitenrändern auf der Substratoberfläche. Die abgesenkten Flanken der Vertiefungen weisen die Silizium <111> Orientierung auf, was dazu führt, dass die vier gegenüberliegenden/benachbarten Flanken der Vertiefung sich in einem atomar scharfen Schnittpunkt treffen (invertierte Pyramiden). Die Grösse der Vertiefungen ist von der Prozessdauer abhängig und kann daher ohne lithographische Verfahren beschränkt kontrolliert werden. Die Dimensionen der derzeitig auch experimentell bestätigten Strukturen liegen bei einer Kantenlänge von 0-400 nm bei entsprechender Tiefe. Das Neue an dem Verfahren ist insbesondere
a) bisher gab es keinen trockenchemischen selektiven Ätzprozess für Silizium;
b) der Ätzprozess ist lokal durch einen Katalysator begrenzt, der den Einsatz von Lithographie ersetzt;
c) Die Dimensionen der Strukturen lassen sich durch die Prozessparameter kontrollieren, konkret durch die Temperatur und die Zeitdauer, in welcher die Temperatur angelegt wird.

Ein ähnlicher Ätz-Mechanismus wird durch flüssige Kalilauge ausgelöst, welche die gleiche Ätzcharakteristik bezüglich der Orientierung der Silizium-Kristalloberflächen besitzt. Allerdings benötigt dieser nasschemische Prozess aufwendige Lithographie-Verfahren, um die Ätzwirkung der Flüssigkeit lokal zu begrenzen, um z.B. Vertiefungen mit vorgegebenen Dimensionen zu erzeugen.

Besonderheiten weisen bevorzugtermassen eingesetzte Gold Katalysatoren auf, die auf eine bestimmte Art und Weise produziert werden, und nur aktiv sind, solange eine gewisse Maximalgrösse (einige Nanometer) nicht überschritten wird. Der Prozess läuft typischerweise bei hohen Temperaturen ab, die dazu führen, dass die katalytisch gelösten Siliziumatome in die Gasphase übergehen.

Eine Silizium-Wafer-Oberfläche kann mit diesem Verfahren grossflächig und homogen strukturiert werden, ohne den Einsatz von stukturbegrenzenden Massnahmen, wie z.B. in Form von Ätzbarrieren in Form von strukturierten Photolacken oder metallischen Ätzmasken, da die Ätzwirkung lokal durch den Katalysator und dessen Positionierung auf dem Substrat begrenzt ist.

Wenn dieses Verfahren auf entsprechend dünnen Silizium-Schichten angewendet wird, deren Dicke etwas geringer als die Tiefe der Pyramiden ist, so können in dem Schnittpunkt der Seitenflanken, am tiefsten Punkt der Pyramiden, wenige Nanometer grosse Öffnungen in einer Silizium-Membran hergestellt werden, so genannte Nanoporen. Diese Nanoporen sind aktuell sehr wichtiger Bestandteil bei der Entwicklung von Nanoporen-basierten Biosensoren, die dazu dienen, DNA-Moleküle elektronisch auszulesen, entsprechende weitere Aspekte betreffend derartige Sensoren werden weiter unten beschrieben werden. Dafür wird ein DNA Molekül durch eine solche Nanopore geführt und beim Passieren der Pore elektronisch ausgelesen.

Ebenfalls kann ein solches Verfahren zur Oberflächenvergrösserung verwendet werden, was ein wichtiger Prozessschritt ist, um die Effizienz von Solarzellen zu steigern.

Als wirtschaftliche und technische Vorteile ergeben sich unter anderem: Sehr unkomplizierte, robuste und kostengünstige Strukturierung und Erzeugung von Nanostrukturen auf ganzen Wafern, gänzlich ohne den Einsatz von lithographischen Methoden (Selbst-Organisation). Prozesstechnische Kontrolle der Dimensionen erlaubt vielseitige Variation, ohne die Anschaffung anfälliger, technisch komplexer und damit teurer Lithographiemasken oder den Einsatz von Elektronenstrahllithographie. Die Erzeugung von geometrisch genau definierten Strukturen im sub 50 nm Bereich, ohne jegliche lithographische Massnahmen, erlaubt die Entwicklung neuartiger elektronischer Bauelemente in parallelisierter Form, auf Waferskala, die ohne diese Methode nur in Form von Einzelprototypen in aufwändigen Laborprozessen herstellbar wären.

Das Verfahren zur trockenchemischen Erzeugung einer Ausnehmung in einem kristallinen Substrat ist bevorzugtermassen dadurch gekennzeichnet, dass auf einer Oberfläche eines kristallinen Substrats an der Stelle der zu erzeugenden Ausnehmung ein Katalysatorpartikel abgelegt wird, und dass, bevorzugtermassen in Anwesenheit einer die Oxidation des Substrats verhindernden Gasatmosphäre, wenigstens der Bereich, auf welchem der Katalysatorpartikel auf der Oberfläche liegt (bevorzugtermassen das gesamte Substrat), während einer Zeitdauer von wenigstens 5 min, vorzugsweise von wenigstens 15 min, einer Temperatur von wenigstens 500 °C, vorzugsweise von wenigstens 750 °C, insbesondere vorzugsweise von wenigstens 900 °C, ganz besonders bevorzugt im Bereich von 900-1100 °C, ausgesetzt wird. Dabei bildet sich eine trichterförmige, sich in die Tiefe des Substrats erstreckende Ausnehmung aus, deren wenigstens drei in die Tiefe des Substrats im Sinne eines Trichters zusammenlaufende Begrenzungsflächen durch Kristallebenen des kristallinen Substrats werden.

Es gibt in diesem Zusammenhang auch eine Herstellungsvariante für solche Nanoporen aus Trichtern in einer dünnen Membran, die sich dadurch auszeichnet, dass die Trichter gerade nicht tiefer sind, als die Membranschicht dick ist. Die letzten paar Nanometer bei einer nicht vollständig durchgeätzten Membranschicht können dabei von der Rückseite her, (der Rückseite der freigelegten und unversehrten Membranoberfläche) durch einen beliebig gearteten Ätzprozess abgetragen werden. Dieses Verfahren hat den Vorteil, dass es damit möglich ist, in einer grossflächigen Membran eine einzige oder eine genau definierte Anzahl von Nanoporen zu erzeugen, indem ein genügend langsamer Ätzvorgang von der Rückseite her genau dann gestoppt wird, wenn die erste (tiefste) Trichteröffnung oder eine genau definierte Anzahl an Trichteröffnungen frei geätzt wurde. Dieser Durchbruch (oder die Durchbrüche) lassen sich z.B. über elektrische oder optische Verfahren detektieren und ermöglichen auf diese Weise das Stoppen eines Ätzvorganges bei einer genau kontrollierten/bevorzugten Anzahl von an der Spitze geöffneten Nanotrichtern.

Zusätzlich kann man das am Ende des Prozesses in den Vertiefungen als Nukleationskeim für ein Silizium Nanodrahtwachstum verwenden, und zwar in einem Verfahren, wie es im Rahmen des ersten Aspekts weiter oben und in den ersten Ansprüchen beschrieben ist. Dies führt zu einer trichterförmigen Struktur mit einem Nanowire, der am tiefsten Punkt angebunden ist, entsprechend betrifft die vorliegende Erfindung auch eine solche neuartige trichterförmige Struktur in einem Silizium-Substrat.

Eine erste bevorzugte Ausführungsform dieses vorgeschlagenen Verfahrens ist dadurch gekennzeichnet, dass es sich beim kristallinen Substrat um ein monokristallines Substrat, bevorzugt aus Silizium handelt, vorzugsweise um einen <100> Silizium-Wafer (beispielsweise eine SOI-Struktur). Wenn ein monokristallines <100> Silizium-Substrat eingesetzt wird, so bildet sich ein Trichter mit vier zusammen laufenden Begrenzungsflächen, die von der Oberfläche in die Tiefe des Substrat zulaufen und durch die <111> Kristallebenen des Einkristalls gebildet werden. Es zeigt sich nämlich, dass der Katalysatorpartikel den Abbau des Silizium entlang dieser Ebenen wesentlich langsamer unter den genannten Bedingungen ablaufen lässt, so dass sich selbsttätig ein derartiger Trichter mit exakt definierten Begrenzungsflächen ausbildet.

Bevorzugtennassen wird die Oberfläche des Substrats, insbesondere des Silizium-Substrats vorher in einer Weise behandelt, dass eine darauf befindliche Oxidschicht entfernt wird.

Eine weitere bevorzugte Ausführungsform ist dadurch gekennzeichnet, dass es sich beim Katalysatorpartikel um einen Metall-Nanopartikel handelt, vorzugsweise um einen Gold-Nanopartikel. Generell verfügt ein solcher Nanopartikel bevorzugtermassen über einen mittleren Durchmesser im Bereich von 1-20 nm, insbesondere vorzugsweise 2-10 nm.

Die Nanopartikel können dabei in unterschiedlicher Weise auf dem Substrat abgelegt werden. Beispielsweise unter Verwendung eines Verfahrens, wie es oben im Zusammenhang mit der Herstellung eines Nanowire beschrieben wurde. Gemäß einer weiteren bevorzugten Ausführungsform wird für die Ablage wenigstens eines Gold-Nanopartikels, insbesondere auf Silizium, so vorgegangen, dass auf der Oberfläche, vorzugsweise unter Verwendung eines Elektronenstrahl-Metallverdampfungsprozesses, bevorzugt im Hochvakuum, eine Goldschicht mit einer Dicke im Bereich von 0.1-2 nm erzeugt wird, aus welcher aufgrund der unterschiedlichen Oberflächenenergie die Gold-Nanopartikel gebildet werden.

Eine weitere bevorzugte Ausführungsform des vorgeschlagenen Verfahrens ist dadurch gekennzeichnet, dass es sich bei der Ausnehmung um eine Durchgangsöffnung durch das Substrat, welches in diesem Fall gewissermassen als Platte mit definierter Schichtdicke ausgebildet ist, handelt, indem die Dicke des Substrats geringer ist als die geometrische, durch die Begrenzungsflächen gebildete Tiefe der Ausnehmung. Dabei weist die Ausnehmung in der Oberfläche des Substrats üblicherweise eine exakte durch die Schnittgeraden zwischen Oberfläche und Begrenzungsflächen gebildete Eingangsöffnung auf und eine durch eine gegenüberliegende unterseitige Oberfläche (oder Übergangsfläche zu einer weiteren anderen Schicht, beispielsweise einer Schicht aus Siliziumdioxid) des Substrats und die Begrenzungsflächen gebildete Ausgangsöffnung mit geringerer, typischerweise geometrisch zur Eingangsöffnung ähnlicher, Querschnittsfläche.

Eine weitere bevorzugte Ausführungsform zeichnet sich dadurch aus, dass die Eingangsöffnung eine rechteckige oder bevorzugtermassen quadratische Querschnittsfläche aufweist mit einer Seitenlänge von 50-500 nm, vorzugsweise im Bereich von 150-250 nm.

Weiterhin bevorzugtermassen verfügt die Vertiefung über eine geometrische, durch die Begrenzungsflächen gebildete Tiefe im Bereich von 50-500 nm, insbesondere bevorzugt im Bereich von 150-250 nm auf, wobei bevorzugtermassen diese geometrische Tiefe 1-50 nm, bevorzugtermassen 5-10 nm größer ist als die Dicke des monokristallinen Substrats, so dass eine Durchgangsöffnung mit einer Ausgangsöffnung ausgebildet wird.

Des weiteren umfasst die vorliegende Erfindung optional ein Verfahren zur Herstellung eines Sensorelements, insbesondere zur Bestimmung von Moleküleigenschaften, insbesondere langkettiger Moleküle, DNA-Moleküle, Polypeptide oder anderer Polymere. Dabei finden bevorzugtermassen die beiden weiter oben beschriebenen Verfahren im Herstellungsprozess Anwendung.

Der elektrische Widerstand eines Silicon Nanowires oder Carbonnanootubes ist sehr sensitiv bezüglich der Veränderungen der elektrischen Ladungen, oder der elektrischen Felder an der Oberfläche der 1-dimensionalen Struktur. Einzelne Elektronenladungen oder einzelne polarisierte Moleküle reichen aus, um die Leitfähigkeit messbar zu verändern. Durch das Entlangziehen eines langkettigen Moleküles (aus verschiedenartigen Bestandteilen DNA/Polypeptiden) an einem solch sensitiven Widerstand kann die Signatur der individuellen Molekülbestandteile durch Widerstandsveränderung ausgelesen werden. Dies kann mit oder ohne vorherige biologische Funktionalisierung der Sensoroberfläche geschehen. Ebenso können die piezoresistiven Eigenschaften von CNTs zur Detektion von Bindungskräften verwendet werden. Durch die Kombination eines 1-dimensionalen elektrischen Leiters (CNT oder Nanowire), direkt über einer Nanopore, ist es möglich, das Molekül durch die Nanopore in lateraler Richtung in der Position zu fixieren. Ein unterschiedliches elektrisches Potential auf den jeweiligen Seiten der Nanopore sorgt dafür, dass z.B. negativ geladene DNA versucht, durch eine Nanopore hindurchzuwandern. Eine Gegenkraft am zurückliegenden Ende des Moleküles ermöglicht es, das Molekül unter mechanischer Spannung mit definierter Geschwindigkeit vor und zurück durch die Pore zu bewegen. Das eine Ende des Moleküls (auf der Seite des Nanowires) kann mittels AFM Tip oder Magnetic Bead/Optical Trap durch die Pore gezogen werden. Dabei wird die Kraft nun so gerichtet, dass die mechanische Spannung das Molekül wie bei einer Seilwinde um den Sensor umlenkt (90°), so wird ein fester mechanischer Kontakt zwischen Molekül und Sensoroberfläche erzeugt. Die Führung durch die Nanopore sorgt für laterale Stabilität und die Erzeugung der benötigten Gegenkraft, um das Molekül in gestrecktem Zustand und in mechanischem Kontakt zum 1D-Leiter aktiv und unter kontrollierter Geschwindigkeit entlang zu scannen.

Der Einsatz von Nanoporen wird derzeit verwendet, um passierende DNA Moleküle auszulesen. Dies geschieht über die Veränderung des Ionenstromes zwischen zwei Reservoirs, die durch eine Nanopore voneinander getrennt sind, während ein DNA-Molekül diese Pore passiert. Diese Nanoporen werden als Einzel-Poren in aufwendiger Prototyp-Arbeit im TEM oder durch Focused Ion-Beam Deposition erzeugt. Probleme bei Nanoporen Sensoren sind dabei u.a.:
i) Aufwändige Herstellung der Nanoporen
ii) Einschluss von Nanobubbles, die den Gebrauch unmöglich machen und viel Rauschen bei der Messung verursachen
iii) Die Moleküle sind sehr schwer durch die ca. 2 nm grosse Pore zu bekommen.
iv) Herstellung und Betrieb ist nur unter Labor-Prototypen Bedingungen möglich

Nanowire- und Nanotube-Sensoren sind an sich bereits realisiert, und haben gezeigt, dass kleinste Bindungs-Kräfte/Ladungsmengen ein messbares Signal erzeugen. Chemische und biologische Funktionalisierung ermöglicht das selektive Binden spezifischer Moleküle und deren Detektion am Sensor. Derzeit ist jedoch noch kein langkettiges Molekül entlang eines solchen Sensors gescannt worden, da es technologisch bisher unmöglich war.

Vorteile des neu vorgestellten Sensorelementes:
a) Nanobubbles sind kein Problem, da nicht in der Pore gemessen wird. Nanobubbles tauchen vermutlich nicht auf, da die Poren grösser 2-10 nm gross sein können.
b) Erstmalig möglich, langkettige Moleküle mit Nanowire/Nanotube Sensoren zu vermessen.
c) In Kombination mit den beiden vorherigen Erfindungsmeldungen ist eine kostengünstige parallele Herstellung solcher Sensoren mit geringem technologischem Aufwand möglich.
d) Paralleler Betrieb mehrerer Sensoren auf einem Chip ermöglicht die parallele Auslesung vieler Moleküle, um die Messgeschwindigkeit zu beschleunigen, was bei langkettigen DNA Molekülen ein kritischer Faktor ist.
e) Sehr kostengünstige Herstellung im Vergleich zu ähnlich komplexen Bauelementen durch Einsatz von grob-auflösender kostengünstiger optischer Lithographie.

Spezifisch umfasst entsprechend die vorliegende Erfindung optional ein Verfahren zur Herstellung eines Sensorelements, insbesondere zur Bestimmung von Moleküleigenschaften von langkettigen Molekülen wie insbesondere DNA-Molekülen oder Polypeptiden, dadurch gekennzeichnet, dass folgende Verfahrensschritte durchlaufen werden, wobei die Reihenfolge gegebenenfalls auch geändert werden kann:
a) in einem Substrat, insbesondere in einer monokristallinen Siliziumschicht, wird eine trichterförmige Durchgangsöffnung mit einer rechteckigen oder quadratischen Eingangsöffnung auf einer Oberseite und einer bezüglich Querschnittsfläche kleineren, bevorzugt wenigstens fünfmal, insbesondere bevorzugt wenigstens zehnmal kleineren, Ausgangsöffnung auf einer der Oberseite gegenüberliegenden Unterseite wird erzeugt, wobei ein Verfahren wie oben beschrieben eingesetzt wird;
b) in einem an die Eingangsöffnung angrenzenden oder nahegelegenen Bereich auf der Oberseite ein Nanowire entweder in einem Verfahren wie oben beschrieben erzeugt und dabei über die Eingangsöffnung und diese überbrückend gelegt und beidseitig über Elektroden kontaktiert wird;
c) die zwei Elektroden in einen Schaltkreis integriert werden, in welchem eine elektrische oder elektronische Eigenschaft über dem Nanowire gemessen werden kann, insbesondere der Widerstand als Funktion der Zeit, insbesondere als Funktion der Position eines ausgemessenen Moleküls.

Gemäß einer ersten bevorzugten Ausführungsform eines solchen Verfahrens ist dieses dadurch gekennzeichnet, dass im Rahmen des Schrittes a) ausgehend von einem Silikon-basierten Gesamtsubstrat mit einer oberflächlichen monokristallinen <100> Siliziumschicht mit einer Dicke im Bereich von 5-500 nm, vorzugsweise im Bereich von 100-300 nm, einer darunter angeordneten Siliziumdioxid-Schicht und einem darunter angeordneten Silizium-Wafer eine trichterförmige Durchgangsöffnung in der Siliziumschicht in einem trockenchemischen oder nasschemischen Ätzverfahren, vorzugsweise in einem Verfahren gemäß der obigen Beschreibung, erzeugt wird, wobei auf der Seite der Siliziumdioxid-Schicht in der Siliziumschicht eine quadratische oder rechteckige Ausgangsöffnung mit einer Seitenlänge im Bereich von 2-10 nm erzeugt wird, indem im Bereich dieser Ausgangsöffnung der Silizium-Wafer und die Siliziumdioxid-Schicht unter Freilegung der Ausgangsöffnung entfernt wird, indem die Siliziumschicht in eine isolierende oxidierte Siliziumdioxidschicht umgewandelt wird. Ebenso kann als Alternative zu dem vollständigen thermischen oxidieren der Siliziummembran eine isolierende Schicht von aussen aufgebracht werden, entweder aus Siliziumdioxid oder einem anders gearteten elektrisch isolierenden Material, welches die Oberfläche der Siliziummembran vollständig bedeckt, aber die Trichterspitzen/Öffnungen offen und durchgängig frei lässt.

Eine weitere bevorzugte Ausführungsform dieses Verfahrens zeichnet sich dadurch aus, dass im Bereich des Sensorelements sowohl auf der Oberseite der Eingangsöffnung als auch auf der Unterseite der Ausgangsöffnung ein Bereich zur Aufnahme einer Flüssigkeit, in welcher zu messende Moleküle gehalten werden können, vorgesehen wird, und wobei zusätzlich Mittel, insbesondere in Form von magnetischen und/oder optischen und/oder elektrooptischen und/oder mechanischen Bewegungselementen vorgesehen werden, mit welchen ein Molekül, welches die Durchgangsöffnung wenigstens teilweise Durchtritt durch diese Durchgangsöffnung hindurch und am Nanowire vorbei und um diesen herum bewegt werden kann, und wobei insbesondere bevorzugt eine Schaltung vorgesehen ist, welche es ermöglicht, eine Potenzialdifferenz zwischen dem Flüssigkeitsbereich auf der Oberseite und dem Flüssigkeitsbereich auf der Unterseite einzustellen.

Des Weiteren umfasst die vorliegende Erfindung optional ein Verfahren zur Herstellung eines Sensorelements nach einem Verfahren wie oben beschrieben, welches dadurch gekennzeichnet ist, dass es ein isolierendes Substrat aufweist mit einer trichterförmigen Durchgangsöffnung mit einer rechteckigen oder quadratischen Eingangsöffnung auf einer Oberseite und einer bezüglich Querschnittsfläche kleineren, bevorzugt wenigstens fünfmal, insbesondere bevorzugt wenigstens zehnmal kleineren, Ausgangsöffnung auf einer der Oberseite gegenüberliegenden Unterseite. Die Trichteröffnung kann nach einem thermischen Umwandlungsprozess in Siliziumdioxid ggf. auch abgeflacht werden und es kann am Ende eine runde Öffnung entstehen, die nicht mehr dem ursprünglichen quadratischen oder rechteckigen Querschnitt entspricht. Dass ein Nanowire über die Eingangsöffnung greifend und diese überbrückend angeordnet ist und beidseitig über Elektroden kontaktiert ist, und diese Elektroden in einen Schaltkreis integriert sind oder werden können, in welchem eine elektrische oder elektronische Eigenschaft über dem Nanowire gemessen werden kann, insbesondere der Widerstand als Funktion der Zeit, insbesondere als Funktion der Position eines ausgemessenen Moleküls.

Vorzugsweise ist dabei sowohl auf der Oberseite der Eingangsöffnung als auch auf der Unterseite der Ausgangsöffnung ein Bereich zur Aufnahme einer Flüssigkeit, in welcher zu messende Moleküle gehalten werden können, vorgesehen wird.

Weiterhin bevorzugtermassen sind zusätzlich Mittel vorgesehen, insbesondere in Form von magnetischen und/oder optischen und/oder elektrooptischen und/oder mechanischen Bewegungselementen, mit welchen ein Molekül, welches die Durchgangsöffnung wenigstens teilweise durchtritt durch diese Durchgangsöffnung hindurch und am Nanowire vorbei und um diesen herum bewegt werden kann.

Weiterhin ist insbesondere bevorzugt eine Schaltung vorgesehen, welche es ermöglicht, eine Potenzialdifferenz zwischen dem Flüssigkeitsbereich auf der Oberseite und dem Flüssigkeitsbereich auf der Unterseite einzustellen.

Des weiteren umfasst die vorliegende Erfindung optional ein Verfahren zur Messung von Eigenschaften von langkettigen Molekülen, insbesondere von DNA-Molekülen oder Polypeptiden, unter Verwendung eines Sensorelements wie oben beschrieben, welches dadurch gekennzeichnet ist, dass das Molekül auf einer Seite mit einem Widerstandselement vorzugsweise in Form eines magnetischen und/oder optisch ansteuerbaren Bead, gekoppelt wird, welches eine Größe aufweist, dass es nicht durch die Durchgangsöffnung hindurch treten kann, und welches durch äußere Einwirkung räumlich relativ zur Durchgangsöffnung lateral zur Oberfläche verschoben werden kann, dass dieses Molekül mit angekoppeltem Widerstandselement in den Flüssigkeitsbereich auf der Oberseite eingetragen wird, dass eine Potenzialdifferenz zwischen dem oberseitigen Flüssigkeitsbereich und dem unterseitigen Flüssigkeitsbereich eingestellt wird, so dass das freie Ende des Molekül durch die Durchgangsöffnung hindurch und in den unterseitigen Flüssigkeitsbereich hindurch gezogen wird, wobei das Widerstandselement im oberseitigen Flüssigkeitsbereich gefangen bleibt, und dass eine äußere Einwirkung, insbesondere in Form einer Laser-single beam trap oder in Form eines magnetischen Feldes, angelegt wird, so dass das Widerstandselement von der Durchgangsöffnung entfernt und/oder angenähert wird, ggf. in einem alternierenden Prozess, wobei die Molekülkette um den Nanowire herum und an diesem kontaktierend vorbeigeführt wird, und dass eine elektrische oder elektronische Eigenschaft, vorzugsweise der sich als Funktion der Zeit ändernde Widerstand, über dem Nanowire gemessen wird.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:

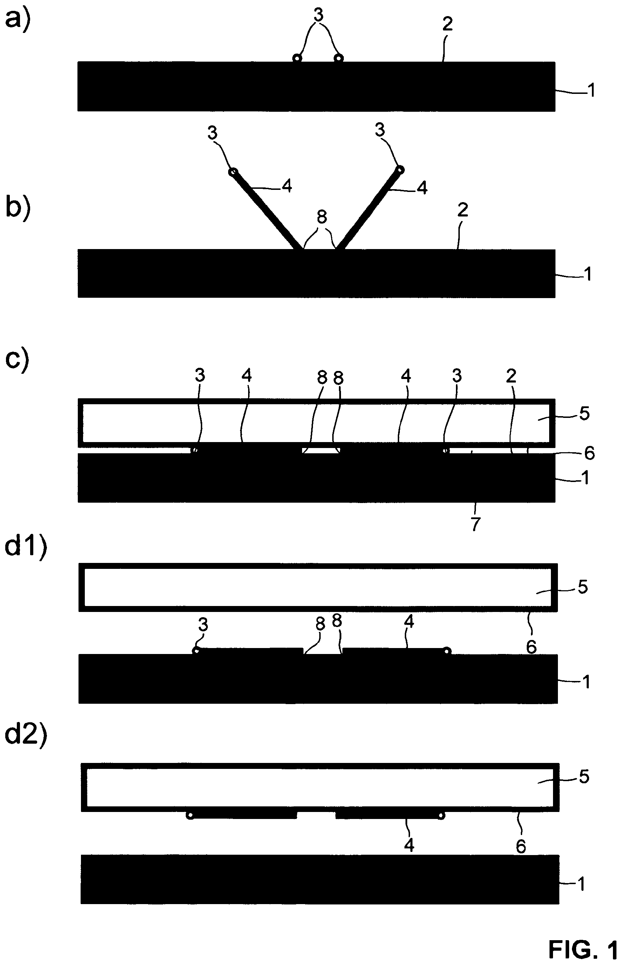

- Fig. 1: Die einzelnen Verfahrensschritte zur Herstellung eines Nanowire in schematischer Darstellung, wobei in a) das Substrat mit darauf abgelagerten Nanopartikeln, in b) darauf gewachsene und von der Oberfläche ab stehende Nanowires, in c) unter Verwendung eines sekundären Substrats auf die Oberfläche geklappte Nanowires dargestellt sind, und wobei in d1) die Situation dargestellt ist, wo das sekundäre Substrat ausschließlich zum herunter klappen der Nanowires ausgelegt ist und wieder entfernt werden kann, und in d2) jene Situation (nicht Teil der Erfindung), wo das sekundäre Substrat adhäsiv ausgebildet ist und die Nanowires auf Letzterem kleben bleiben;

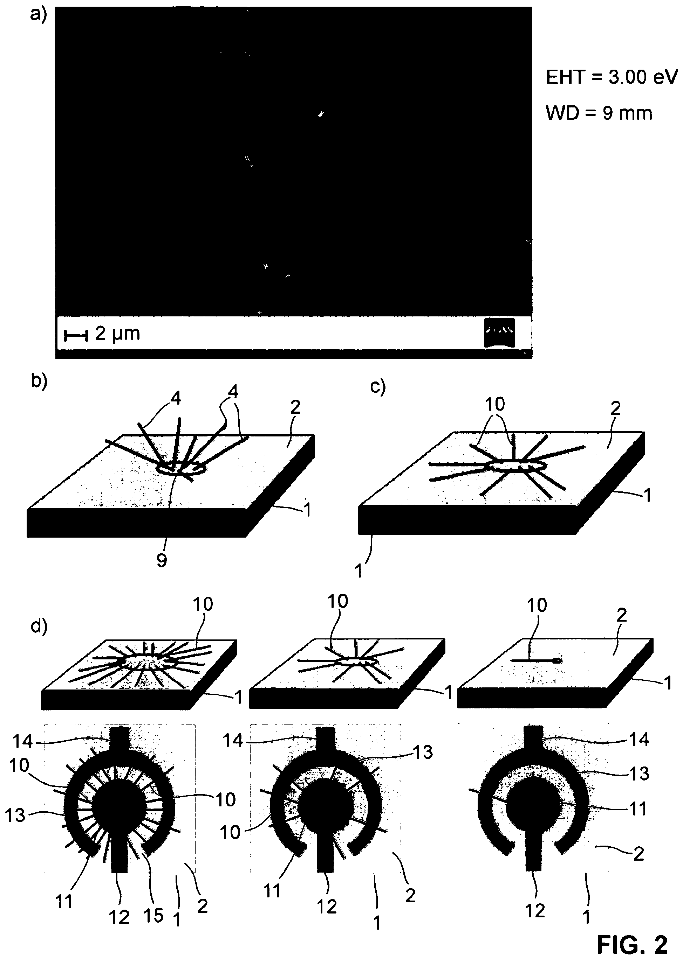

- Fig. 2: in a) eine elektronenmikroskopische Aufnahme von Gruppen von Nanowires auf einem Substrat, in b) schematisch eine Gruppe von von der Ebene ab stehenden Nanowires auf einem Nukleationsareal, in c) die Gruppe gemäß Figur b) nachdem die Nanowire herunter geklappt wurden, in d) verschiedene Darstellungen von Nukleationsarealen mit unterschiedlicher Anzahl von Nanowires, jeweils oben in perspektivische Ansichten und darunter entsprechende Aufsichten auf mit Elektroden kontaktierte Nanowires für diese unterschiedlichen Belegungen;

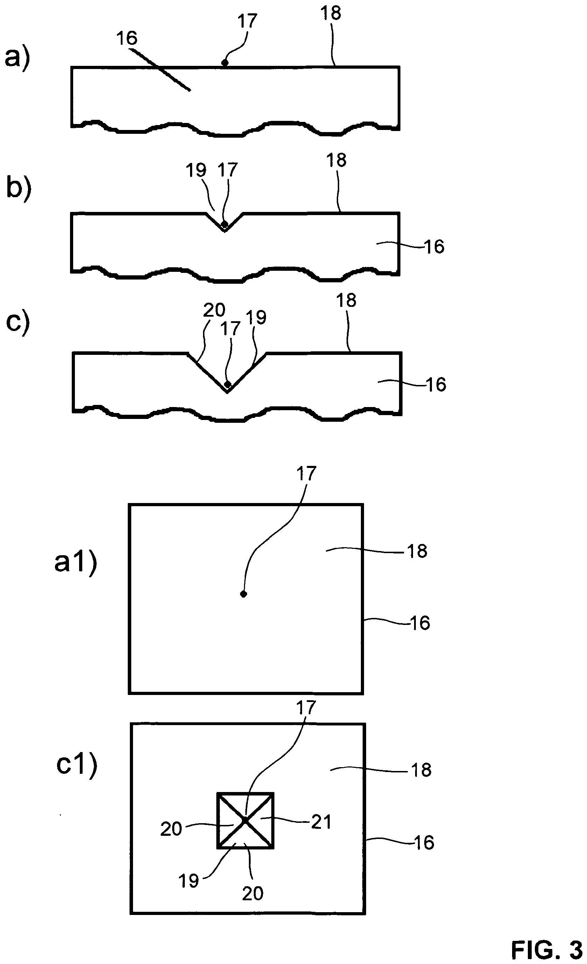

- Fig. 3: die unterschiedlichen Verfahrensschritte bei der Herstellung eines Trichters in einem Siliziumsubstrat, wobei in a) eine schematische Schnittdarstellung gegeben ist mit darauf liegenden Goldpartikel, in a1) eine Aufsicht auf a), in b) eine Darstellung in einem Schnitt nach einem ersten Trichterwachstum, in c) eine Darstellung in einem Schnitt nach einem tiefen Trichterwachstum, und in c1) eine Aufsicht auf die Situation gemäß c);

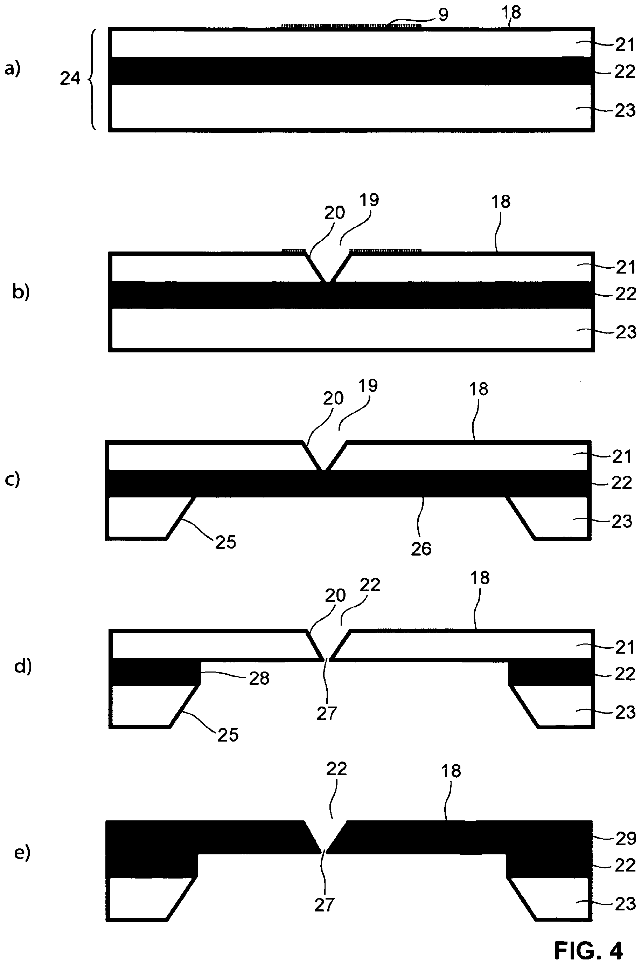

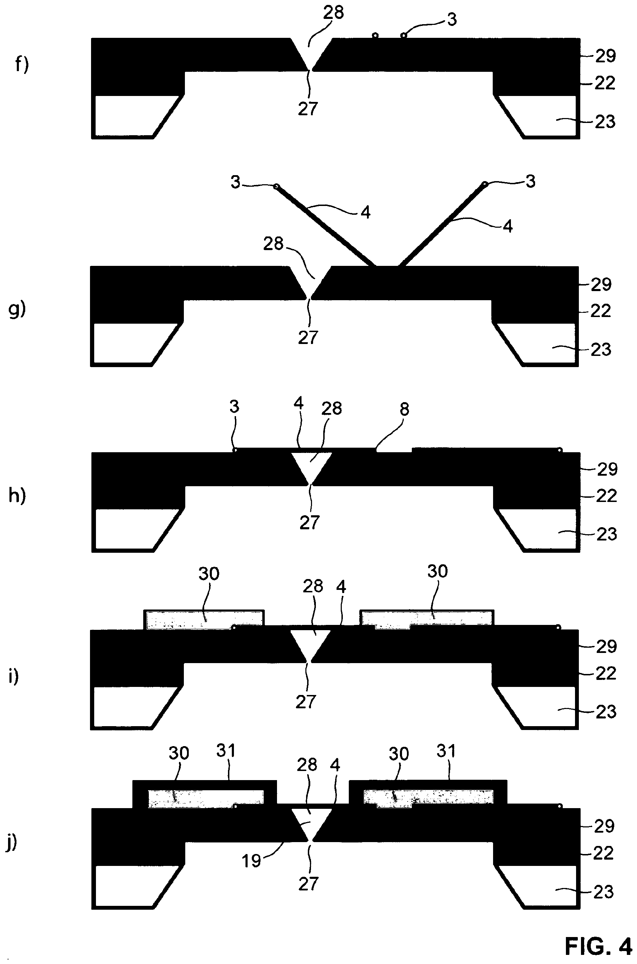

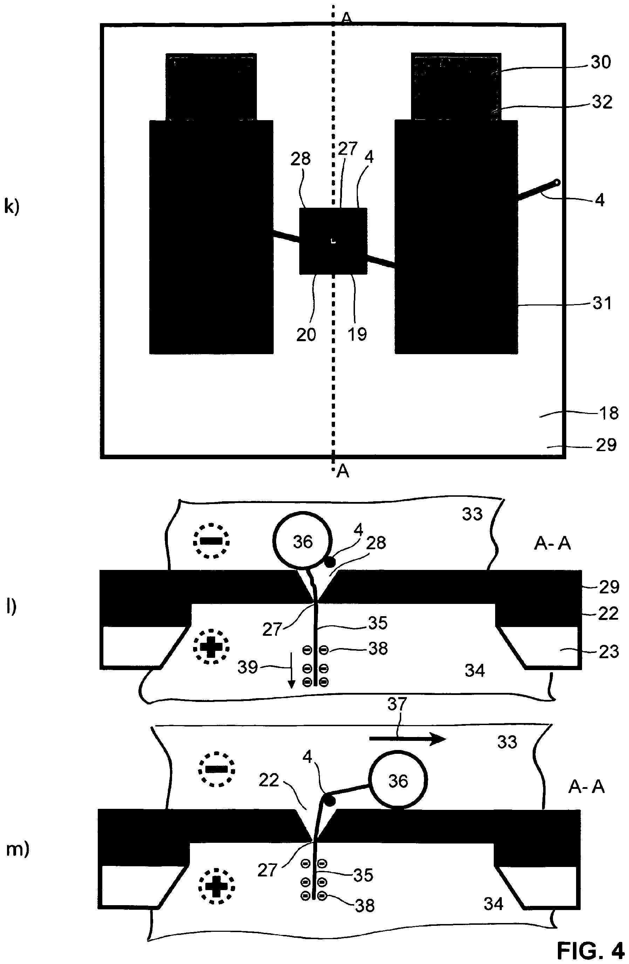

- Fig. 4: die einzelnen Schritte der Herstellung eines Sensors in ihrer zeitlichen Abfolge und in schematischer Schnittdarstellungen das Messprinzip, wobei a) ein SOI Substrat zeigt, auf welchem mittels optischer Lithographie definierte Fläche mit einer 0.5 nm Au Aufdampfung versehen wurde, in b) das Resultat des bei 950°C gefahrenen Si-Ätzprozess dargestellt ist, welches statistisch verteilt Nano-Trichter in der vorher definierten Fläche erzeugt, in c) das Resultat der Schritte der Entfernung der Gold-Reste und der Rückseitenätzung des Si-Wafers mit KOH, wobei die Oberseite für diesen Prozessschritt bevorzugtermassen vor der Kalilauge geschützt werden sollte man beispielsweise durch Schutzlack, in d) das Resultat der Entfernung der SiO2 Schicht mittels HF, in e) das Resultat der Umwandlung der frei schwebenden Membran in einem thermischen Oxidationsschritt in SiO2 um die gewünschte elektrische Isolation zu gewährleisten, in f) das Resultat der Definition mittels optischer Lithographie der Orte für die Deposition der Gold-Katalysatorpartikel, wobei die Goldpartikel werden dort deponiert sind, in g) die gewachsenen Nanowires auf der Oberfläche, wobei die Goldpartikel lokal das Wachstum von Si-Nanodrähten katalysieren und wobei durch das amorphe SiO2 Substrat die Orientierung willkürlich ist, in h) das Resultat der lateralen Immobilisierung der Nanowires auf dem Silizium Oxid-SubstratMit einem nicht-adhäsivem Hilfssubstrat, in i) die Kontaktierung der Enden der Nanowires, welche über dem Trichter liegen, mittels optischer Lithographie, mit Metallelektroden, in j) Überzug der Metallelektroden mittels optischer Lithographie mit einer isolierenden Schicht aus SiO2 oder Si-Nitrid, in k) eine Aufsicht auf den resultierenden Sensor, wobei ein Teil der Metallelektroden für die Integration in Messelektronik frei bleibt , und wobei der freiliegende Teil der Metallelektroden ausserhalb der Flüssigkeitskammer liegt, in der sich die zu untersuchenden Moleküle befinden, und wobei die Nanowires werden gegebenenfalls chemisch funktionalisiert sind, um gezielt Bindungsmöglichkeiten mit speziellen Basen der DNA/oder anderen molekularen Bausteinen zu ermöglichen, in l) eine Schnittdarstellung entlang der gestrichelten Linie in k) durch einen Sensor, wobei die Anordnung wird in eine Flüssigkeitskammer eingebettet, in der sich die DNA (o.ä.) Moleküle befinden, und wobei die DNA ist mit Polymerkügelchen oder magnetischen Kugeln funktionalisiert, wobei zusätzlich Elektroden in die Flüssigkeitskammer eingetaucht werden, um ein elektrostatisches Potential zwischen den separierten Reservoirs zu erzeugen, wobei mittels optischen Fallen oder einem magnetischen Feld die Moleküle über die angehängten Kugeln zu den Trichtern transportiert werden, und wobei das elektrostatische Potential zieht die DNA in die Trichter und das Molekül spannt, und in m) stellt dar, wie mittels optischer Fallen oder einem magnetischen Feld, das relativ zum Substrat bewegt wird (oder umgekehrt) die Moleküle an den Kügelchen parallel zum Substrat entlang der Nanodrähte gezogen werden, wobei die elektronische Signatur über die Nanodrähte ausgelesen wird;

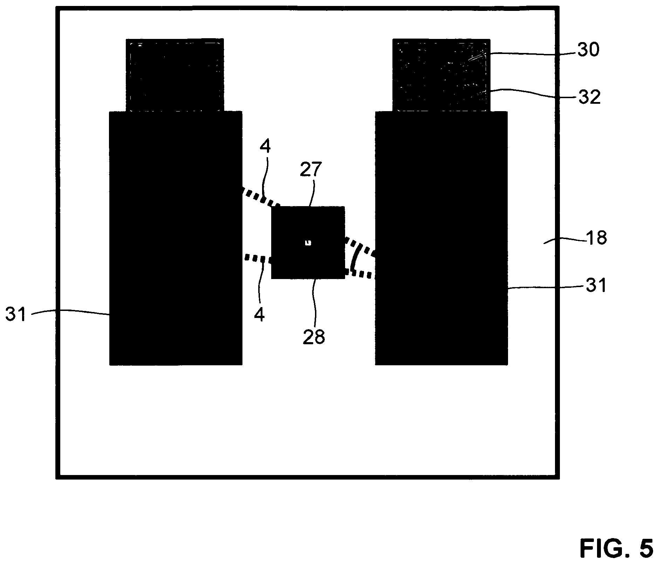

- Fig. 5: in einer Aufsicht eine Darstellung analog zu Figur 4k), wobei unterschiedlich verteilte Nanowires dargestellt sind über dem gleichen Nano-Trichter.

### BESCHREIBUNG BEVORZUGTER AUSFÜHRUNGSFORMEN

Gemäß einem ersten Aspekt der vorliegenden Erfindung betrifft diese ein Verfahren zur Herstellung von Nanowire-Strukturen in einer elektrischen Schaltung. In der Folge soll dieser Aspekt in detaillierterer Form beschrieben werden, namentlich Verfahren für die Herstellung von large scale Arrays elektrisch kontaktierter Nanowires in lateraler Ausrichtung auf einem iosolierenden Substrat vorgestellt werden.

Das Verfahren ermöglicht die effiziente Kontaktierung einzelner oder einer begrenzten Vielzahl von Nanodrähten mit Metallelektroden, zum Zweck, zum Beispiel Biosensoren in large scale Arrays herzustellen, wie dies im Rahmen des dritten Aspekts bereits erläutert wurde und weiter unten im Detail dargelegt werden wird.

Hintergrundinformationen zum vapor-liquid-solid (VLS) Nanowire Wachstumsprozess:
Silizium Nanowires können mittels Katalysatorpartikeln, welche auf einem Substrat immobilisiert sind, lokal gewachsen werden. In einem Gasphasen-Abscheidungsprozess kommt es, lokal am Ort der Katalysatorpatikel, zu einem 1-dimensionalen faserartigen Kristallwachstum. Dabei wird ein Silizium enthaltendes Gas, nur am Ort des Katalysators, von der Gasphase in einen Festkörper umgewandelt. Der Durchmesser der Kristallfasern ist im wesentlichen durch den Durchmesser der Katalysatorpartikel definiert. Jedes Katalysatorteilchen erzeugt maximal eine Kristallfaser (Nanowire). Geeignete Katalysatorpartikel sind für Silizium: Gold, TiO₂, Aluminium und eine weitere. Für die verschiedenen Katalysatormaterialien gibt es unterschiedliche optimale Prozesstemperaturen. Mit Gold lassen sich typischerweise zwischen 325°C und > 1000°C Nanowires wachsen. Für andere Metalle gelten ggf. höhere Mindesttemperaturen.

Die Länge der Kristallfasern skaliert im wesentlichen linear mit der Prozessdauer.

Nanowires können auf jedem beliebigen Substrat gewachsen werden, welches die gewählte Prozesstemperatur unbeeinflusst übersteht. Der hier gewählte Prozess läuft bei ca. 465°C ab. Das ist knapp unterhalb der Temperaturgrenze, jenseits welcher es zu unkatalytischer Siliziumdeposition auf dem gesamten Substrat kommt.

Detaillierter Verfahrensablauf (vergleiche auch Figur 1):
Man nehme ein Substrat 1, zum Beispiel eines mit einer elektrisch isolierenden Oberfläche 2, z.B. oxidiertes Silizium oder ein Glassubstrat.

Auf diesem Substrat 1 wird, ggf. nach einer Reinigung zum Beispiel unter Verwendung der RCA Standardreinigungsmethode, mittels optischer Lithographie ein Photolack aufgebracht, der an den gewünschten Stellen für das Nanowirewachstum Löcher enthält. Dafür wird das Substrat mit einem lichtempfindlichen Photolack überzogen. Dieser Lackfilm wird durch selektive Belichtung über eine strukturierte Chrom-Maske, welche sich auf einem transparenten Quartzglas-Träger befindet, mit den gewünschten Strukturen versehen. Der Prozess ist ein Prozess, für den sich viele verschieden Lacke mit vielen verschiedenen Belichtungsparametern und Schichtdicken eignen.

Im vorliegenden Fall können es Löcher, mit 0.02- 10 Mikrometern Durchmesser sein. Für viele Anwendungen eignen sich 0.5-5 Mikrometer.

Die Löcher im Lack, die sich an den gewünschten Stellen befinden, dienen nun dazu, selektiv an den Stellen, wo das darunter liegende Substrat frei zugänglich ist, Gold-Katalysatorpartikel zu deponieren.

Es gibt dafür zwei mögliche Prozesse, die beide zu gleichen/ähnlichen Resultaten führen:
a) Eine wässrige Lösung, welche Gold Nanopartikel (Kolloide) in gewünschter Grösse enthält, wird auf den Lack und das Substrat aufgebracht. Über reine Physisorption lagern sich die Gold Kolloide in den Löchern auf dem SiO2 oder Glas Substrat ab. Die wässrige Lösung evaporiert nach einigen Stunden und es bleiben die Gold Kolloide nun immobilisiert auf der Oberfläche zurück. Ein geeignetes Lösungsmittel (Aceton) entfernt dann den Lack und die darauf immobilisierten Gold Kolloide. Es bleiben lediglich Gold Kolloide an den Stellen auf dem Substrat zurück, wo das Substrat durch Löcher im Lack frei zugänglich war (sogenannter Lift-Off Prozess).
   Es gibt unterschiedlichste Grössen von Gold Nanopartikeln in wässriger Lösung zwischen 0.5 und 500 Nanometern. Es wurden im Rahmen der experimentellen Überprüfung der Erfindung 6-8 verschiedene Lösungen mit Partikeln der Grössen insbesondere zwischen 5 nm bis 150 nm ausprobiert: dabei stellt sich heraus, dass sich überraschenderweise lediglich die Gold Kolloide mit 40 nm Durchmesser sich auf dem Substrat in den Löchern ablagern. Alle anderen Grössen dringen aus irgendwelchen, noch nicht erkannten Gründen, nicht in die Löcher ein. Dadurch lassen sich über die Strukturierung mittels Photolack und Lift-Off kleine Gold Kolloide strukturiert auf das Substrat aufbringen. Die Feststellung der selektiv abgelegten und funktionierenden ca. 40 nm Partikel für diesen Prozess wird im Moment noch nicht vollständig verstanden. Ohne durch diese Erklärung gebunden zu sein, so scheint es im Moment so zu sein, dass ein physikalischer Grund dafür sein könnte, dass die Gold Kolloide elektrostatischer Wechselwirkung mit dem Photolack unterliegen und wegen Abstossungsgründen nicht in die Löcher eindringen. Die ca. 40 nm Gold Kolloide haben eventuell durch die Herstellung bedingt, eine andere Oberflächenchemie/Oberflächenladung und unterscheiden sich daher von den übrigen Partikeln. Das könnte ein Grund dafür sein, warum die Kombination von optischer Lithographie und wässrigen Lösungen von Gold Kolloiden bisher wenig Beachtung für strukturiertes Wachstum von Silizium Nanodrähten gefunden hat.
b) Ein zweiter Prozess eignet sich ebenfalls, um über die Vorstrukturierung eines Photolackes Katalysatoren auf das Substrat aufzubringen. Mittels Elektronenstrahl-Metallverdampfung (im Vakuum) lässt sich ein dünner Goldfilm auf die strukturierte Lackschicht und das in den Löchern freiliegende Substrat aufbringen. Bei der Aufdampfung von Goldschichten mit einer nominalen Schichtdicke von 0.1 - 2 nm bilden sich, auf dem in den Löchern freiliegenden Substrat, Gold Nanopartikel aus. Dieses Verfahren ist an sich bereits bekannt, es wird diesbezüglich verwiesen auf Albuschies, J., M. Baus, O. Winkler, B. Hadam, B. Spangenberg, and H. Kurz, High-density silicon nanowire growth from self-assembled Au nanoparticles. Microelectronic Engineering, 2006. 83(4-9): p. 1530-1533, die in dieser Publikation genannte detailliertere Beschreibung des Verfahrens ist entsprechend in die vorliegende Beschreibung mit eingeschlossen.

Der Lack und das darauf befindliche Gold werden mittels eines geeigneten Lösungsmittels (beispielsweise hier spezifisch Aceton) vom Substrat entfernt. Lediglich, dort wo die Löcher im Lack waren, bleiben auf dem darunter liegenden Substrat Gold Nanopartikel immobilisiert (Lift-Off Prozess).

Die nun strukturiert aufgebrachten Gold Nanopartikel 3, die resultierende Struktur ist schematisch in Figur 1a) dargestellt, dienen zum lokalen Wachstum von Silizium Nanodrähten 4 auf dem Substrat 1 (die Situation nach dem Wachstum ist schematisch in Figur 1 b) dargestellt.

Die Nanodrähte 4 haben einen Durchmesser, der im wesentlichen von den individuellen Katalysatorpartikeln 3 vorgegeben wird. Da das darunter liegende Substrat 1 amorph ist, gibt es keine vorgegebene Wachstumsrichtung (Orientierung) der Silizium Kristallfasern in Bezug zum Substrat.

Auf dem Bereich, wo sich die Goldpartikel befunden haben, gibt es nach dem Wachstumsprozess eine Vielzahl beliebig orientierter Silizium Nanodrähte, welche eine nicht genau quantifizierte Winkelverteilung im gesamten Halbraum oberhalb des Substrates einnehmen (wie ein halbierter "Seeigel", der auf dem Substrat liegt, vergleiche auch Figur 2a) respektive b)).

Der Verankerungspunkt 8 der Nanodrähte 4 auf dem Substrat 1 liegt dort, wo sich ursprünglich der Katalysator-Metallpartikel befunden hat. Je nach Länge der Nanodrähte 4 reichen die Nanodrähte 4 in lateraler Richtung weit über den Ort hinaus, wo die Nanodrähte 4 auf dem Substrat 1 verankert sind.

Um die Nanodrähte 4 elektrisch mit Metallelektroden 11/13 kontaktieren zu können, ist eine laterale (parallele) Ausrichtung der Nanodrähte zum Substrat 1 notwendig.

Die Nanodrähte 4 müssen für diesen Prozess parallel auf dem elektrisch isolierenden Substrat 1 liegen. Essentiell für den Prozess ist, dass Ort und Orientierung der Nanodrähte 4 bekannt sein müssen, um in einem folgenden Schritt Metallelektroden 11/13 aufbringen zu können. Beide Enden eines Nanowires 4 müssen dafür zwischen zwei unterschiedlichen Elektroden 11/13 verbunden sein. Die Nanowires 4 stellen demnach die einzige elektrische Verbindung zwischen zwei makroskopischen Elektroden 11/13 dar, welche die Einbindung eines einzelnen oder wenigen Nanodrähten 4 in einen grösseren Schaltkreis ermöglicht.

Da der Ort der Katalysatorpartikel über die optische Lithographie definiert und daher bekannt ist, muss nur noch die Orientierung bestimmt werden.

Alignmentmarker können vor dem Prozess auf das Substrat aufgebracht werden und definieren ein Koordinatensystem. Relativ zu diesem Koordinatensystem lassen sich zuerst die Katalysatorpartikel aufbringen und definieren daher einen Ort für die Nanowires 4. Wenn die Orientierung bekannt ist, dann können mit Hilfe des Koordinatensystems auf dem Substrat die Metallelektroden 11/13 für die jeweiligen Nanowires 4 auf das Substrat 1 aufgebracht werden.

Der Ausrichtevorgang um die Nanodrähte 4 parallel auf das Substrat 1 zu bekommen basiert auf der mechanischen Kraftausübung auf die Nanodrähte 4 und die daraus resultierende permanente Änderung der Orientierungsrichtung der Nanodrähte 4, wie dies schematisch in den Figuren 1 c)-d) dargestellt ist.

Ein Hilfsmittel 5 mit einer gewissen Geometrie wird benutzt, um eine Kraft auf die Nanodrähte 4 auszuüben, so dass die Nanodrähte 4, welche nicht parallel zum Substrat 1 orientiert sind, nach der Kraftausübung flach auf dem Substrat 1, d.h. parallel zu dessen Oberfläche 2, liegen.

Eine (vektorielle) Komponente der Kraft muss dafür senkrecht in Richtung Substrat 1 wirken. Diese Kraft kann über eine flache oder gewölbte Fläche eines sekundären Substrats 5 ausgeübt werden.

Im Fall eines flachen Hilfsmittels werden die Nanodrähte alle gleichzeitig auf das Substrat gedrückt (Sandwich). Im Falle einer gewölbten Fläche werden die Nanodrähte sequentiell auf das Substrat gedrückt (wie bei einer Teigrolle/Nudelholz). Eine Kugel, die über das Substrat rollt, ist ebenfalls möglich.

Die Nanodrähte 4 bleiben, vermutlich im wesentlichen aufgrund von van-derWaals Kräften, auf dem Substrat immobilisiert. Eine spezielle adhäsive Oberflächenfunktionalisierung auf Substrat und Nanowires kann den Prozess effizienter machen. So beispielsweise die Verwendung eines Klebers, der nicht auf dem Hilfsmittel haftet, aber Nanowires und Substrat fest miteinander verbindet.

Um zu verhindern, dass die Nanodrähte an dem kraftausübenden Hilfsmittel 5 haften bleiben (Situation gemäß Figur 1d1), kann eine spezielle Anti-Haft-Beschichtung auf dem Hilfsmittel 5 vorgesehen werden, die weniger Wechselwirkungen mit den Nanodrähten 4 hat, als das Substrat 1, auf dem die Nanodrähte 4 immobilisiert werden sollen. Der Prozess funktioniert jedoch in vielen Fällen ohne Kleber oder ohne Anti-Haft-Beschichtung des Hilfsmittels 5.

In hier durchgeführten Experiment wurde ein Mask-Aligner (sonst für die Lithographie verwendet) als technischer Apparat benutzt, der es ermöglicht das Nanowire-Substrat von unten her gegen eine Quartzglasscheibe (Hilfsmittel 5) zu drücken. Ein solcher Apparat ist besonders geeignet, da es zu keiner (oder sehr geringer) lateraler Verschiebung zwischen Nanowire-Substrat und Quartzglasscheibe während Annäherung und Entfernung kommt.

Wenn der Durchmesser der anfänglichen Katalysator-Depositionsfläche 9 klein ist, im Verhältnis zur Länge der Nanodrähte, dann sind die Nanodrähte nach dem Ausrichtevorgang radial zum Ort der Katalysatorfläche 2 orientiert, wie dies beispielsweise anhand von Figur 2c) erkannt werden kann.

Mit dem Wissen um die radiale Anordnung und mit dem bekannten Mittelpunkt der Anordnung lassen sich präzise die Metallelektroden auf die Nanodrähte aufbringen, wie dies in Figur 2 d) illustriert ist.

Die Elektroden 11-14 können über Lithographie und Elektronenstrahl-Metallverdampfung und anschliessendem Lift-Off Prozess hergestellt werden.

Um die Anordnung in einem flüssigen Medium zu betreiben ist üblicherweise noch eine Passivierung/elektrische Isolierung der Metallelektroden zur Umgebung vorzunehmen, damit die Metallelektroden nicht über das flüssige Medium elektrisch leitend verbunden werden können.

Detaillierte Beschreibung des Prozesses zur Herstellung der Nanowires:
Die Gold Katalysatoren (5-50 nm) wird auf einem Substrat 1 aufgebracht, welches die hier gewählten Prozesstemperaturen von 465°C aushält. Die Substrate 1 können für die vorgeschlagenen Anwendungen Silizium, Siliziumdioxid, oder herkömmliches Glas sein.

Die Substrate 1 werden in einen Ofen gegeben, der normalerweise zur Gasphasenabscheidung verwendet wird. Das ist in diesem Fall ein von aussen beheiztes Quartzglasrohr, bei welchem der Innendruck genau kontrolliert werden kann.

Bei der Prozesstemperatur von 465°C wird ein Gasgemisch aus Silan und Wasserstoff in die Prozesskammer gegeben. Disilan und andere Silizium enthaltenen Gase sind ist ebenfalls möglich (SiH₄, Si₂H₆).

Der Druck wird während des Prozesses auf 5 Millibar reduziert. Da das Siliziumenthaltende Gas während des Prozesses verbraucht wird, wird ein kontinuierlicher Gasfluss durch die Prozesskammer gewährleistet. Die Gasmengen für den speziellen Prozess sind: 100 sccm (Standardkubikzentimeter) Silan und 200 sccm Wasserstoff.

Wasserstoff ist dabei ein Trägergas, welches keine direkte chemische katalytische Funktion besitzt, und kann durch ein anderes Gas wie beispielsweise Stickstoff ersetzt werden. Der Prozess funktioniert auch mit reinem Silan, oder in der Gegenwart anderer inerter Gase.

Die Prozessdauer liegt typischerweise bei 30-60 Minuten, wobei die Prozessdauer direkt proportional mit der Länge der resultierenden Nanodrähte 4 zusammenhängt. Die Drähte wachsen über die Prozessdauer mit einer vermutlich im wesentlichen konstanten Wachstumsgeschwindigkeit. Bei den angegebenen Parametern ergibt sich eine Wachstumsgeschwindigkeit von etwa 10-20 Mikrometern pro Stunde. Der Durchmesser der Nanowires entspricht in etwa der Grösse der Goldpartikel (5-50 nm). Zu Beginn des Prozesses gibt es eine Verzögerungsphase während der im wesentlichen kein Wachstum stattfindet. Das Wachstum setzt erst nach einigen Minuten ein (vermutlich zunächst Aktivierung und Sättigung der Katalysatorpartikel, bis sich das zum Wachstum ideale eutektische Gemisch zwischen Gold und Silizium gebildet hat).

Der Silandruck (oder Partialdruck in Gegenwart anderer Gase) und die Temperatur bestimmen die Wachstumsgeschwindigkeit. Höherer Druck, als auch höhere Temperaturen führen zu schnellerem Wachstum.

Wichtige bei diesem Prozess ist, dass keine unkatalytische Siliziumdeposition vom Gas auf das Substrat stattfindet. Ansonsten wird das ganze Substrat (auch zwischen den Katalysatorpartikeln) mit einer amorphen Siliziumschicht überzogen und die elektrisch isolierenden Eigenschaften z.B. eines SiO₂ Substrates werden negativ beeinflusst.

Die unkatalytische Deposition von Silizium aus Silangas beginnt bei ca. 470°C aufwärts und ist relativ substratunabhängig.

Typische Parameter: Substrat: SiO2/Glas; Katalysatoren: Gold Nanopartikel (5-50 nm); Prozesstemperatur :465°C; Prozessdauer: 30-60 Minuten; Gasfluss: 100 sccm Silan, 200 sccm Wasserstoff; Gesamtdruck: 5 mbar.

Wie oben beschrieben umfasst
die vorliegende Erfindung daneben gemäß einem weiteren Aspekt zudem ein trockenchemisches Verfahren zur Oberflächenbearbeitung von monokristallinen Substraten. Dieses Verfahren soll nun insbesondere unter Zuhilfenahme von Figur 3 im Detail beschrieben werden im Rahmen von experimentellen Vorschriften.

Das Verfahren für die lithographiefreie Herstellung von pyramidenförmigen Vertiefungen in einkristallinem Silizium mit Hilfe eines neuen trockenchemischen Ätzverfahrens lässt sich wie folgt durchführen:
Das Substrat 16 ist ein gereinigter Siliziumwafer mit der <100> Kristallorientierung in der Substratoberfläche 18. Der Wafer sollte oxidfrei sein und wird daher vor dem Prozess mit Flusssäure von der natürlichen Oxidschicht befreit werden:
1 Minute im HF Bad (hydrofluoric acid). 10 Minuten spülen mit deionisiertem Wasser.(DIH₂O). Der Schritt zur Entfernung des natürlichen Oxides ist aber nicht zwingend notwenig. Die ca. 2 nm dicke natürliche Oxidschicht wird während des Prozesses durch die Ätzwirkung des Goldes überwunden und stellt keine definitiv sichere Ätzbarriere zwischen Gold und Silizium dar.

Danach wird eine Goldschicht in einem Hochvakuum mittels Elektronenstrahl-Metallverdampfung aufgebracht. Die nominale Schichtdicke beträgt 0.1-2.0 Nanometer.

Die geringe nominale Schichtdicke führt dazu, dass sich kein geschlossener Goldfilm bildet, sondern dass sich nanometer-grosse Gold (Au) Cluster 17 auf der Siliziumoberfläche 18 bilden (vergleiche Figur 3 a). Es können auch andere Methoden zum aufbringen von Au Nanopartikeln 17 für den Prozess geeignet sind, beispielsweise Prozesse, wie sie oben im Rahmen des ersten Aspekts der Erfindung beschrieben wurden.

Für die Formierung von Au Nanopartikeln 17 ist die unterschiedliche Oberflächenenergie zwischen Gold und Silizium verantwortlich. (Auf SiO₂ z.B. werden die Partikel bei gleicher nominaler Schichtdicke etwa 2-5 mal so gross, wie auf Si).

Der Siliziumwafer 16 mit den aufgebrachten Goldpartikeln 17 wird bei 950°C bei Normaldruck in einem Quarts-Rohr-Ofen unter Stickstoffatmosphäre oder einem anderen Inert-Gas aufgeheizt (es ist lediglich eine Oxidation des Siliziums zu verhindern).

Es entstehen zunächst atomar kleine quadratische Vertiefungen 19, die über die Prozesszeit in ihren Dimensionen proportional wachsen (vergleiche Figur 3 b). Die Prozesszeit definiert die Grösse/Tiefe der pyramidenförmigen Vertiefungen 19. Nach 30 Minuten bei 950° haben die Vertiefungen ca. 200 nm Durchmesser. Die Tiefe entspricht den unter 57.x° abgesenkten Flanken der Vertiefungen, entsprechend der Pyramidenform. Die Flanken 20, die die Silizium <111> Kristalloberfläche besitzen, treffen (schneiden) sich im Zentrum der Vertiefung 19 und bilden eine atomar scharfe Falte.

Obwohl die Siliziumoberfläche homogen mit Metall bedampft wurde, und dadurch die Gold Nanopartikel 17 eine sehr hohe Dichte (Anzahl pro Fläche) aufweisen, bilden sich die geätzten Vertiefungen in einer wesentlich geringeren Anzahl pro Flächeneinheit.

Ohne dass einer solchen theoretischen Erklärung einschränkender Charakter zugemessen werden soll, so wird im Moment doch vermutet, dass bei der Bedampfung mit dem Gold eine statistisch verteilte Vielzahl an verschieden grossen Gold-Nanopartikeln entsteht, und dass in der Hauptsache eine ganz bestimmte Grösse der Gold Nanopartikel 17 das katalytische Ätzen des Siliziums ermöglicht. Die Verteilung der geätzten Vertiefungen 19 entspräche in diesem Fall exakt der Verteilung der geeigneten Goldpartikel 17 auf der Siliziumoberfläche. Ebenso ist es aber dadurch erklärbar, dass Oberflächendefekte der Kristallstruktur in der Siliziumoberfläche 18 als Nukleationskeime für den Ätzprozess agieren. In dem Fall wäre die Dichte der Pyramidenlöcher substrat- und nicht partikelabhängig.

Für die weitere Verwendung der nanostrukturierten Siliziumoberflächen kann das überschüssige Gold mittels Kaliumjodid oder anderen gold-lösenden Prozessen entfernt werden (z.B. Königswasser).

Zusätzlich kann man das Gold 17 in den Vertiefungen 20 als Nukleationskeim für ein Silizium Nanodrahtwachstum verwenden, und zwar in einem Verfahren, wie es im Rahmen des ersten Aspekts weiter oben und in den ersten Ansprüchen beschrieben ist. Man bekommt damit Silizium Nanodrähte, die aus den pyramidenförmigen Löchern herauswachsen.

Herstellung von Nanotrichtem in einer dünnen Siliziummembran (vergleiche diesbezüglich auch die Figuren 4 a)-c)):
Man nehme einen kommerziell erwerblichen SOI Wafer mit der <100> Kristallorientierung als Silizium Top Layer: SOI = Silicon on Insulator.

Ein SOI Wafer 23 ist ein herkömmlicher Siliziumwafer mit normaler Dicke. Jedoch befinden sich auf der einen Seite des Wafers eine dünne Siliziumoxidschicht 22 und darüber eine weitere dünne monokristalline Siliziumschicht 21. Bei dieser Anordnung befindet sich das Oxid 22 zwischen dem Wafer 23 und der dünnen Siliziumschicht 21.

Geeignete Dimensionen für die Schichtdicken sind:
Für die Silizium-Top-Schicht 21: 5-500 nm. Die Dicke der darunterliegenden Oxidschicht spielt eine untergeordnete Rolle. Sie muss bevorzugtermassen dick genug sein, um den Au-Ätzvorgang als Ätzbarriere stoppen zu können (typischerweise > 5-10 nm).

Der oben beschriebene trockenchemische Ätzvorgang wird nun auf dem SOI Wafer ausgeführt. Die Dimenionen, im Speziellen die Tiefe der pyramidenförmigen Vertiefungen 19, werden so angestrebt (über die Prozessdauer bei 950°), dass die Tiefe der resultierenden Löcher/Pyramiden (die geometrische aus den Begrenzungsflächen definierte Pyramide) eine von Null verschiedene Anzahl von Nanometern tiefer wären, als die Top-Siliziumschicht an Dicke besitzt. Beispiel 200 nm Silicon on Insulator und 205 nm tiefe Pyramiden. Das hat zur Folge, dass der Ätzvorgang bei einer Tiefe von 200 nm an der Oxidschicht terminiert wird und Pyramiden mit abgeschnittenen Spitzen entstehen.

Es bildet sich am Boden der Pyramiden 19 in einer Tiefe von 200 nm ein Plateau aus der darunter liegenden SiO₂ Schicht 22.

Die Grösse des Plateaus lässt sich sehr genau einstellen. Dies geschieht über die Differenz zwischen nominaler Pyramidentiefe und vorhandener Dicke des Silizium Toplayers 21.

Der SOI Wafer kann nun von der Unterseite her mittels herkömmlichen nasschemischen Ätzverfahren so strukturiert werden, dass die darunter liegende dicke Siliziumschicht und ebenfalls die SiO2 Schicht entfernt werden (vergleiche Darstellungen gemäß Figur 4 b)-e) und entsprechende detailliertere Beschreibung weiter unten). Es bleibt dann nur die Ursprüngliche Silizium Topschicht 21 als frei schwebende Membran zurück. Silizium wird über Kalilauge entfernt und die SiO₂ Schicht über Flussäure. Der Silizium Top-Layer wird zu diesem Zweck mittels eines Kalilauge- und HF-resistenten Schutzfilm aus einem Polymermaterial geschützt. Dort, wo die pyramidenförmigen Vertiefungen (an ihrer tiefsten Stelle) das Plateau aus SiO₂ besessen haben, befinden sich nun, der Grösse des Plateaus, entsprechend grosse Öffnungen. (Pore/Loch/Appertur).

Die Si 23 und die SiO₂ Schicht 22 auf der Unterseite des SOI Wafers kann ebenfalls strukturiert (lokal) entfernt werden, so dass der Grossteil des Wafers als mechnischer Support für die frei schwebende dünne Silizium Topschicht 21 dient. Die Unterseite des Wafers wird zu diesem Zweck mit optischer Lithographie strukturiert. Die Öffnungen in dem Fotolack können einige Mikrometer bis mehrere Millimeter gross sein, je nach Anforderung an die Grösse/Fläche der frei schwebenden und mit Trichtern versehenen dünnen Siliziummembran.

Wie oben beschrieben umfasst die vorliegende Erfindung optional auch ein Verfahren zur Herstellung eines Sensorselementes, wobei dieses Gebrauch macht von den oben beschriebenen Verfahren zur Erzeugung eines Nanowire respektive zur Herstellung eines trichterförmigen Loches in einer Silizium-Membran. In einer Alternative, die nicht zur Erfindung gehört, kann aber anstelle eines Nanowire beispielsweise ein CNT eingesetzt werden, und die trichterförmigen Öffnung kann auch unter Verwendung eines nasschemischen Verfahrens in der Silizium-Membran erzeugt werden. Das Verfahren zur Herstellung soll in der Folge unter Zuhilfenahme von Figur 4 a)-k) dargelegt werden, und die Betriebsweise unter Zuhilfenahme von Figur 4 l)-m).

Nanowire Sensor mit Nanotrichtern zur Analyse langkettiger Moleküle, Sensorgeometrie und die Betriebsweise (vergleiche Figur 4):
Der Sensor besteht aus einem elektrisch kontaktierten Siliziumnanodraht 4 oder einem Kohlenstoffnanoröhrchen (Carbon Nanotube, CNT), welcher sich auf einem isolierenden Substrat (einer isolierenden Membran 18/29) befindet. Unter den Nanodraht 4 (oder CNT) befindet sich ein Nanotrichter 19, der eine Öffnung in dem Substrat/Membran zur Unterseite darstellt. Die zu analysierenden Moleküle 35 sind langkettige Polymere/Polypeptide, welche aus unterschiedlichen Untereinheiten aufgebaut sind (Proteine/DNA). Die elektrische Sensitivität des Nanodrahtes 4 (CNT) erlaubt es, die einzelnen Untereinheiten des langkettigen Moleküls 35 bei direktem physikalischem Kontakt zwischen beiden zu detektieren und elektronisch über den Nanodraht/CNT 4 auszumessen. Zur Sequenzierung aller Untereinheiten wird das Molekül der Länge nach über den Nanodraht/CNT 4 gezogen und liefert damit sequentiell die elektrischen Informationen, die den einzelnen Molekularen Bausteinen entsprechen.

Zur Verbesserung der elektrische Detektion der molekularen Untereinheiten kann der Nanodraht/CNT 4 speziell chemisch funktionalisiert werden, um eine stärkere Wechselwirkung zwischen den einzelnen Untereinheiten und dem Nanodraht 4 zu erzeugen, wodurch die Signale der einzelnen molekularen Bausteine verstärkt werden. Im Falle von DNA können die komplementären Basenpaare auf die Nanowires/CNTs aufgebracht werden, um durch die Bindung eine grösseres elektrisches Signal zu erzeugen.

Es gibt dabei insbesondere zwei mögliche Betriebsweisen:
A) Die Wechselwirkung kann über die elektrostatische und molekülspezifische Widerstandsänderung eines Nanodrahtes/CNTs detektiert werden (wie bei der Detektion kleiner Moleküle).
B) Ebenso ist es möglich, den piezoresistiven Effekt eines Nanodrahtes/CNTs zu nutzen, um die molekularen Bausteine zu identifizieren. Der elektrische Widerstand eines Nanowires/CNTs ändert sich mit einer auf den Nanodraht/CNTs ausgeübten Kraft und der daraus resultierenden Ausdehnung/Längenänderung dessen. Die molekularen Untereinheiten binden je nach Funktionalisierung des Nanowires/CNTs unterschiedlich stark an den Nanodraht/CNT und üben beim sequentiellen Entlangziehen unterschiedliche Kräfte auf den Nanodraht/CNT aus, welche dann elektrisch ausgelesen werden können.

Die spezielle Geometrie des Nanowire-Sensors mit Trichter über einer Membran erlaubt es, das Molekül genau über dem Nanowire zu positionieren und genau gerichtete Zugkräfte/Zuggeschwindigkeiten bei einer genau definierten Umlenkung des Moleküls über den Nanodraht/CNT auszuüben.

Um die gerichteten Kräfte auf das Molekül ausüben zu können und um das Molekül über den Sensor positionieren zu können, werden die Moleküle 35 an einem Ende mit einem Polymer-Kügelchen 36 funktionalisiert, das 0.5-10 Mikrometer im Durchmesser gross ist, so dass es nicht durch den Trichter 19 passt. Ebenfalls dient das Polymerkügelchen 36 dazu, die einzelnen Moleküle mit Hilfe einer optischen Falle, oder im Falle eines magnetischen Kügelchens mit einer magnetischen Falle, zu bewegen und über den Trichter 19 zu positionieren (Optical Trap = fokussierter Lichtstrahl, mit dem man kleine Objekte in Flüssigkeit bewegen kann). DNA kann z.B. spezifisch an den jeweiligen Enden mit verschiedensten Objekten/Polymer-Beads chemisch verbunden werden.

Die starke negative elektrische Ladung 38 der DNA erlaubt es, das Molekül zur Unterseite, d.h. aus dem Flüssigkeitsbereich 33 in den Flüssigkeitsbereich 34 entlang der schematisch dargestellt Richtung 39 durch den Trichter 19 zu ziehen. Dies geschieht mittels eines elektrischen Potentials zwischen den Flüssigkeitsbereichen 33/34 auf der Ober- und Unterseite der Membran 29, in der sich die Trichteröffnung 19 befindet. Das elektrostatische Potential wird über Elektroden in der Flüssigkeit jeweils ober- und unterhalb der isolierenden Membran 29 erzeugt. Das Molekül 35 würde ohne das Polymerkügelchen durch die Trichteröffnung gezogen werden.

Das Molekül erfährt nun eine elektrostatische Zugkraft 39 zur Unterseite: Diese Kraft ist senkrecht zur Membran ausgerichtet. Mit Hilfe einer optischen Falle kann nun über das Polymerkügelchen 36 eine laterale Zugkraft auf das Molekül 35 ausgeübt werden, die der elektrostatischen Zugkraft 39 entgegengerichtet ist. Dadurch wird das Molekül 35 gespannt und übt eine mechanische Kraft durch die Umlenkung um den Nanodraht auf denselben aus. Zudem ist aufgrund der kleinen Dimensionen der Austrittsöffnung 27 das Molekül relativ zum Draht 4 in seiner Position exakt festgelegt. Mit Hilfe der optischen Falle kann die Geschwindigkeit des Entlangziehens genau kontrolliert werden und in beide Richtungen reversibel hin und her geschaltet werden. Das ermöglicht mehrfaches Auslesen zur Verminderung des Mess-Rauschens.

Mittlerweile sind optische Geräte erhältlich, die aus einer einzigen Laserquelle mittels Akkusto-Optischen-Strahl-Teilern bis zu hunderte optische Fallen auf einem Substrat unabhängig voneinander positionieren und bewegen können. Dadurch lässt sich eine parallele Betriebsweise von mehreren Sensoren auf einem Substrat realisieren (parallele DNA Sequenzierung).

Herstellungsverfahren (vergleiche Figur 4 a)-k) und entsprechende Figurenbeschreibung): Genau wie bei der separaten oben bereits erfolgten Beschreibung zur Herstellung der Nanotrichter in Membranen wird ein SOI Wafer 23/24 genommen.

Mittels optischer Lithographie werden auf der Top-Silizium Schicht 21 die Flächen definiert, wo sich die Trichter 19 befinden sollen. Auf diese Flächen wird die nominale Schichtdicke von 0.5 nm Gold aufgedampft, was zur Schicht 9 in klar definierten Bereichen auf der Oberfläche 18 führt. Der Heizprozess bei 950°C und der entsprechenden Zeit liefert die Nanotrichter 19. Wie separat beschrieben, wird von der Unterseite her der Siliziumwafer mittels Kalilauge entfernt und die SiO₂ Schicht mittels Flussäure weggeätzt. Die nun frei schwebende Silizium Membran 21 wird in einem thermischen Oxidierungsschritt in elektrisch isolierendes SiO₂ zur Schicht 29 umgewandelt.

Auf der SiO₂ Membran 29 mit den Nanotrichtem 19 werden nun an den gewünschten Orten mittels optischer Lithographie die Flächen definiert, auf denen die Gold Katalysatorpartikel 3 zum Wachstum der Nanodrähte 4 liegen sollen. Die Flächen für das Nanodrahtwachstum befinden sich direkt neben den Flächen auf denen die Nanotrichter 19 erzeugt wurden. Der Nanodraht-Wachstumsprozess wird, wie separat beschrieben, ausgeführt.

Die unausgerichteten Nanodrähte 4 werden über das Umklappverfahren lateral auf das Substrat 29 immobilisiert. Ein Teil der Nanodrähte 4 wird sich wie gewünscht über den Nanotrichtern 19 befinden, ein gewisser Anteil jedoch nicht. Die Verteilung von gut positionierten Nanowires über Trichtern ist statistisch und kann je nach Nanowiredichte und Trichterdichte bis zu einem gewissen Grad variiert werden. Dies ist aber unproblematisch, weil die Eingangsöffnung des Trichters genügend gross ist (vergleiche auch Figur 5). Durch die grosse Anzahl an Bauelementen 19/4, die parallel auf einem Substrat 29 hergestellt werden können, ist auch ein relativ geringer Yield von gut positionierten Nanodrähten 4 ausreichend, um auf einem Chip eine ausreichend grosse Anzahl an gut positionierten Nanodrähten 4 zu erreichen.

Die lateral positionierten Nanodrähte 4 werden dann mit Metallelektroden 30 über optische Lithographie kontaktiert. Um die Sensoranordnung in einer elektrolytischen Flüssigkeit 33/34 betreiben zu können müssen die Metallelektroden 30 elektrisch von der Umgebung isoliert werden. Dazu werden die Metallelektroden 30 mit einer elektrisch isolierenden Schicht 31 aus SiO₂ oder Siliziumnitrid überzogen, wobei die Nanodrähte 4 frei bleiben (ebenfalls mittels optischer Lithographie).

Zum betreiben des Sensors wird die Anordnung in eine mikroskopietaugliche Kammer eingebettet, so dass zwei voneinander getrennte Flüssigkeitsreservoirs 33/34 entstehen, die nur durch die Löcher 19 der Membranen 29 miteinander verbunden sind. In den getrennten Reservoirs 33/34 befinden sich zusätzliche Elektroden um die elektrostatische Kraft auf die DNA Moleküle auszuüben und durch die Trichter 19 zu ziehen.

Mittels optischem Mikroskop und optischen Fallen können die Beads 36 mit der DNA 35 über den geeigneten Sensoren positioniert werden, um die DNA 35 an den Nanowires 4 entlang zu ziehen und elektrisch auszulesen.

**BEZUGSZEICHENLISTE**

| | | | |
|---|---|---|---|
| 1 | Substrat, primäres Substrat | 22 | Siliziumoxid-Schicht |
| 2 | Substratoberfläche | 23 | Silizium-Wafer |
| 3 | Nanopartikel, Gold-Partikel | 24 | Gesamtsubstrat |
| 4 | Nanowire | 25 | unterseitige Ausnehmung in 23 nach Behandlung mit Kalilauge |
| 5 | sekundäres Substrat, Hilfsmittel | | |
| 6 | Oberfläche von 5 | 26 | freigelegte Oberfläche von 22 |
| 7 | Spalt | 27 | Ausgangsöffnung von 19 |
| 8 | Anbindungspunkt von 4 an 2 | 28 | Eingangsöffnung von 19 |
| 9 | Bereich mit Nanopartikeln, Nukleationsareal | 29 | oxidierte Schicht 21, Siliziumoxid-Schicht als |
| 10 | flach gelegte Nanowires | | isolierende Substratschicht |
| 11 | zentraler Elektrodenbereich | 30 | Metallelektrode |
| 12 | Anschluss von 11 | 31 | Isolationsschicht |
| 13 | teilweise umlaufende zweite Elektrode | 32 | Kontaktbereich von 30 |
| | | 33 | Flüssigkeit auf Oberseite |
| 14 | Anschluss von 13 | 34 | Flüssigkeit auf Unterseite |
| 15 | Aussparung in 13 für 12 | 35 | Molekül-Strang |
| 16 | Siliziumsubstrat | 36 | Bead, Polymerkugel |
| 17 | Nanopartikel, Gold-Partikel | 37 | laterale Bewegung von 36 |
| 18 | Oberfläche von 16 | 38 | Ladungen an 35 |
| 19 | Ausnehmung, Trichter in 16 | 39 | Zugkraft an 35 wegen 38 und Potenzialdifferenz zwischen 33 und 34 |
| 20 | Begrenzungsfläche von 19, Kristallebene | | |
| 21 | Siliziumschicht | 40 | Unterseite von 21/29 |

## Patentansprüche

1. Verfahren zur Herstellung einer Leiterstruktur mit wenigstens einem Nanowire, insbesondere einem Silizium-Nanowire (4), mit einem Durchmesser von weniger als 50 nm, der über wenigstens zwei Stellen über Elektroden (11,13, 30) kontaktiert ist, und wobei der wenigstens eine Nanowire (4) und die Elektroden (11, 13,30) in einer Ebene auf einem Substrat (1, 29) angeordnet sind, wobei
a) auf der Oberfläche (2) eines isolierenden Substrats (1, 29) katalytisch aktive Metall-Nanopartikel mit einem Durchmesser im Bereich von 0.5-50 nm auf lokal definierten Nukleationsflächen (9) abgelegt werden,
b) bei einer Temperatur im Bereich von 300-1100 °C während einer Zeitdauer im Bereich von 10-200 min die Oberfläche und die darauf abgelagerten Metall-Nanopartikel einem Gasstrom enthaltend wenigstens eine gasförmige Siliziumkomponente ausgesetzt werden, wobei sich wenigstens ein vom Substrat (1) abstehender Nanowire (4) einer Länge im Bereich von 5-200 µm bildet;
c) dieser wenigstens eine von der Oberfläche des Substrats (1) abstehende Nanowire (4) durch Auflegen eines Sekundärsubstrats (5) mit einer der Oberfläche (2) des isolierenden Substrats (1, 29) korrespondierenden Kontaktfläche (6) in eine Ebene abgelegt wird;
d) und der auf dem isolierenden Substrat (1, 29) abgelegte wenigstens eine Nanowire (4) an zwei unterschiedlichen Stellen mit Elektroden (11, 13,30) kontaktiert wird, wozu eine erste Elektrode (11) über dem Zentrum der entsprechenden Nukleationsfläche (9) und eine zweite Elektrode (13) in ringförmiger Anordnung um die erste Elektrode (11) erzeugt werden.

2. Verfahren nach Anspruch 1, **dadurch gekennzeichnet, dass** es sich beim isolierenden Substrat (1) um ein Substrat aus Silizium, Siliziumdioxid, Siliziumnitrid oder Glas handelt, und dass es sich bei den Metall-Nanopartikeln um Gold-Nanopartikel handelt, vorzugsweise mit einem Durchmesser im Bereich von 5-50 nm, vorzugsweise im Bereich von 20-45 nm.

3. Verfahren nach einem der vorhergehenden Ansprüche, **dadurch gekennzeichnet, dass** im Rahmen des Schrittes a) die Metall-Nanopartikel in räumlich begrenzten und voneinander getrennten Bereichen auf dem Substrat abgelegt werden, bevorzugtermassen indem eine Schicht aus einem Fotolack, insbesondere unter Verwendung von optischer Lithographie, aufgebracht wird, wobei, bevorzugt über selektive Belichtung über eine strukturierte Chrommaske, Löcher mit einem Durchmesser im Bereich von 0.02-10 µm, vorzugsweise 0.5-5 µm, in dieser Schicht aus Fotolack erzeugt werden, und indem
entweder anschließend eine bevorzugt wässrige Lösung, welche Metall-Nanopartikel als Kolloide trägt, vorzugsweise Nanopartikel mit einem Durchmesser im Bereich von 0.5-500 nm, insbesondere vorzugsweise mit einem Durchmesser im Bereich von 5-150 nm, auf den Fotolack aufgebracht wird, wobei die Lösung abgedampft wird und wobei anschließend der Lack mit einem geeigneten Lösungsmittel, bevorzugt Aceton, entfernt wird,
oder indem unter Verwendung von Elektronenstrahl-Metallverdampfung im Vakuum ein Metallfilm, insbesondere ein Goldfilm, auf diese Schicht aus Fotolack aufgebracht wird, vorzugsweise mit einer Schichtdicke im Bereich von 0.1-2 nm, und indem anschließend der Fotolack und das darauf befindliche Metall mittels eines geeigneten Lösungsmittels, bevorzugt Aceton, entfernt wird.

4. Verfahren nach einem der vorhergehenden Ansprüche, **dadurch gekennzeichnet, dass** im Schritt b) bei einer Temperatur im Bereich von 350-500 °C, vorzugsweise im Bereich von 400-480 °C, insbesondere vorzugsweise im Bereich von 450-470 °C gearbeitet wird, und indem als gasförmige Siliziumkomponente ein Silan oder ein Disilan, vorzugsweise in Kombination mit einem Trägergas, insbesondere bevorzugt Stickstoff oder Wasserstoff, eingesetzt wird, wobei bevorzugtermassen ein Gasfluss im Bereich von 50-200 sscm vom Silan respektive Disilan eingesetzt wird und ein Gasfluss im Bereich von 100-300 sscm Trägergas, und indem ein Gesamtdruck im Bereich von 1-50 mbar, vorzugsweise im Bereich von 2-10 mbar über dem Substrat gehalten wird.

5. Verfahren nach einem der vorhergehenden Ansprüche, **dadurch gekennzeichnet, dass** im Schritt a) die Nanopartikel in wenigstens einem, vorzugsweise in mehreren, voneinander getrennten, Nukleationsarealen (9) abgelegt werden, so dass sich im Schritt b) eine Mehrzahl von Nanowires (4) über jedem Nukleationsareal (9) ausbildet, und dass im Rahmen des Schrittes d) eine erste zentrale Elektrode (11) über dem Nukleationsareal (9) ein erstes Ende der Nanowires kontaktierend, vorzugsweise über eine Metallbedampfung oder eine fotolithographische Deposition, erzeugt wird, und eine zweite, wenigstens teilweise die erste Elektrode (11) umlaufend ausgebildete zweite Elektrode (13), vorzugsweise über eine Metallbedampfung oder eine fotolithographische Deposition, erzeugt wird.

6. Verfahren zur Herstellung eines Sensorelements, insbesondere zur Bestimmung von Moleküleigenschaften, **dadurch gekennzeichnet, dass**
a) in dem Substrat (21), insbesondere in einer monokristallinen Siliziumschicht, eine trichterförmige Durchgangsöffnung (19) mit einer rechteckigen oder quadratischen Eingangsöffnung (28) auf einer Oberseite (18) und einer bezüglich Querschnittsfläche kleineren, bevorzugt wenigstens fünfmal, insbesondere bevorzugt wenigstens zehnmal kleineren, Ausgangsöffnung (27) auf einer der Oberseite (18) gegenüberliegenden Unterseite (40);
b) in einem an die Eingangsöffnung (28) angrenzenden oder nahegelegenen Bereich auf der Oberseite (18) ein Nanowire (4) in einem Verfahren gemäß einem der Ansprüche 1-5 erzeugt und dabei über die Eingangsöffnung (28) und diese überbrückend gelegt wird und beidseitig über Elektroden (30) kontaktiert wird;
c) die zwei Elektroden (30) in einen Schaltkreis integriert werden, in welchem eine elektrische oder elektronische Eigenschaft über dem Nanowire (4) gemessen werden kann, insbesondere der Widerstand als Funktion der Zeit, insbesondere als Funktion der Position eines ausgemessenen Moleküls (35).

7. Verfahren nach Anspruch 6, **dadurch gekennzeichnet, dass** im Rahmen des Schrittes a) des Anspruchs 6 ausgehend von einem Silikon-basierten Gesamtsubstrat (24) mit einer oberflächlichen monokristallinen <100> Siliziumschicht (21) mit einer Dicke im Bereich von 5-500 nm, vorzugsweise im Bereich von 100-300 nm, einer darunter angeordneten Siliziumdioxid-Schicht (22) und einem darunter angeordneten Silizium-Wafer (23) eine trichterförmige Durchgangsöffnung in der Siliziumschicht (21) in einem trockenchemischen oder nasschemischen Ätzverfahren erzeugt wird, wobei auf der Seite der Siliziumdioxid-Schicht (22) in der Siliziumschicht (21) eine quadratische oder rechteckige Ausgangsöffnung (27) mit einer Seitenlänge im Bereich von 2-10 nm erzeugt wird, indem im Bereich dieser Ausgangsöffnung (27) der Silizium-Wafer (23) und die Siliziumdioxid-Schicht (22) unter Freilegung der Ausgangsöffnung (27) entfernt wird, indem die Siliziumschicht (21) in eine isolierende oxidierte Siliziumdioxidschicht (29) umgewandelt wird.

8. Verfahren nach einem der Ansprüche 6 oder 7, **dadurch gekennzeichnet, dass** im Bereich des Sensorelements sowohl auf der Oberseite der Eingangsöffnung (28) als auch auf der Unterseite der Ausgangsöffnung (27) ein Bereich zur Aufnahme einer Flüssigkeit, in welcher zu messende Moleküle gehalten werden können, vorgesehen wird, und wobei zusätzlich Mittel, insbesondere in Form von magnetischen und/oder optischen und/oder elektrooptischen und/oder mechanischen Bewegungselementen vorgesehen werden, mit welchen ein Molekül (35), welches die Durchgangsöffnung (19) wenigstens teilweise Durchtritt durch diese Durchgangsöffnung (19) hindurch und am Nanowire (4) vorbei und um diesen herum bewegt werden kann, und wobei insbesondere bevorzugt eine Schaltung vorgesehen ist, welche es ermöglicht, eine Potenzialdifferenz zwischen dem Flüssigkeitsbereich auf der Oberseite und dem Flüssigkeitsbereich auf der Unterseite einzustellen.

9. Verfahren nach einem der Ansprüche 6 - 8, wobei das Substrat (16) ein kristallines Substrat ist und bei Schritt a) des Anspruchs 6 ein Verfahren zur trockenchemischen Erzeugung einer Ausnehmung (19) in einem kristallinen Substrat (16) verwendet wird, **dadurch gekennzeichnet, dass** auf einer Oberfläche (18) des kristallinen Substrats (16) an der Stelle der zu erzeugenden Ausnehmung (19) ein Katalysatorpartikel (17) abgelegt wird, und dass in Anwesenheit einer die Oxidation des Substrats (16) verhindernden Gasatmosphäre wenigstens der Bereich, auf welchem der Katalysatorpartikel (17) auf der Oberfläche (18) liegt, während einer Zeitdauer von wenigstens 5 min, vorzugsweise von wenigstens 15 min, einer Temperatur von wenigstens 500 °C, vorzugsweise von wenigstens 750 °C, insbesondere vorzugsweise von wenigstens 900 °C, ganz besonders bevorzugt im Bereich von 900-1100 °C, ausgesetzt wird, wobei sich eine trichterförmige Ausnehmung (19) ausbildet, deren wenigstens drei in die Tiefe des Substrats zusammenlaufende Begrenzungsflächen (20) durch Kristallebenen des kristallinen Substrats (16) gebildet werden.

10. Verfahren nach Anspruch 9, **dadurch gekennzeichnet, dass** es sich beim Substrat (16) um ein monokristallines Substrat, bevorzugt aus Silizium handelt, vorzugsweise um einen (100) Silizium-Wafer, insbesondere um eine SOI-Struktur, wobei es sich insbesondere bevorzugt im Letzteren Fall bei den Begrenzungsflächen (20) um vier von der Oberfläche in die Tiefe des Substrat zulaufende (111) Kristallebenen des Einkristalls handelt.

11. Verfahren nach einem der Ansprüche 9-10, **dadurch gekennzeichnet, dass** es sich beim Katalysatorpartikel (17) um einen Metall-Nanopartikel handelt, vorzugsweise um einen Gold-Nanopartikel (17), vorzugsweise mit einem Durchmesser im Bereich von 1-20 nm, insbesondere vorzugsweise 2-10 nm, wobei bevorzugtermassen die Gold-Nanopartikel (17) auf der Oberfläche (18) erzeugt werden, indem auf der Oberfläche, vorzugsweise unter Verwendung eines Elektronenstrahl-Metallverdampfungsprozesses, bevorzugt im Hochvakuum, eine Goldschicht mit einer Dicke im Bereich von 0.1-2 nm erzeugt wird, aus welcher aufgrund der unterschiedlichen Oberflächenenergie die Gold-Nanopartikel (17) gebildet werden.

12. Verfahren nach einem der Ansprüche 9-11, **dadurch gekennzeichnet, dass** es sich bei der Ausnehmung (19) um eine Durchgangsöffnung durch das Substrat (16) handelt, indem die Dicke des Substrats (16) geringer ist als die geometrische, durch die Begrenzungsflächen (20) gebildete Tiefe der Ausnehmung (19) und wobei die Ausnehmung in der Oberfläche (18) des Substrats eine durch die Schnittgeraden zwischen Oberfläche (18) und Begrenzungsflächen (20) gebildete Eingangsöffnung (28) aufweist und eine durch eine gegenüberliegende unterseitige Oberfläche des Substrats (16) und die Begrenzungsflächen (20) gebildete Ausgangsöffnung (27) mit geringerer, geometrisch zur Eingangsöffnung (28) ähnlicher Querschnittsfläche.

13. Verfahren nach Anspruch 12, **dadurch gekennzeichnet, dass** die Eingangsöffnung (28) eine polygonale, bevorzugt rechteckige oder quadratische, Querschnittsfläche aufweist mit einer Seitenlänge von 50-500 nm, vorzugsweise im Bereich von 150-250 nm, und dass die Vertiefungen bevorzugtermassen eine geometrische, durch die Begrenzungsflächen (20) gebildete Tiefe im Bereich von 50-500 nm, insbesondere bevorzugt im Bereich von 150-250 nm aufweisen, wobei bevorzugtermassen die geometrische Tiefe 1-50 nm, bevorzugtermassen 5-10 nm größer ist als die Dicke des Substrats, so dass eine Durchgangsöffnung mit einer Ausgangsöffnung (27) ausgebildet wird.

14. Verfahren gemäß einem der Ansprüche 6-13, **dadurch gekennzeichnet, dass** ein Sensorelement hergestellt wird, das ein isolierendes Substrat (29) aufweist mit einer trichterförmigen Durchgangsöffnung (19) mit einer, bevorzugt rechteckigen oder quadratischen, Eingangsöffnung (28) auf einer Oberseite (18) und einer bezüglich Querschnittsfläche kleineren, bevorzugt wenigstens fünfmal, insbesondere bevorzugt wenigstens zehnmal kleineren, Ausgangsöffnung (27) auf einer der Oberseite (18) gegenüberliegenden Unterseite (40), dass der wenigstens eine Nanowire (4) über die Eingangsöffnung (28) greifend und diese überbrückend angeordnet ist und beidseitig über Elektroden (30) kontaktiert ist, und diese Elektroden (30) in einen Schaltkreis integriert sind oder werden können, in welchem eine elektrische oder elektronische Eigenschaft über dem Nanowire (4) gemessen werden kann, insbesondere der Widerstand als Funktion der Zeit, insbesondere als Funktion der Position eines ausgemessenen Moleküls (35), wobei vorzugsweise sowohl auf der Oberseite der Eingangsöffnung (28) als auch auf der Unterseite der Ausgangsöffnung (27) ein Bereich zur Aufnahme einer Flüssigkeit, in welcher zu messende Moleküle gehalten werden können, vorgesehen wird, und weiterhin bevorzugtermassen wobei zusätzlich Mittel, insbesondere in Form von magnetischen und/oder optischen und/oder elektrooptischen und/oder mechanischen Bewegungselementen vorgesehen werden, mit welchen ein Molekül (35), welches die Durchgangsöffnung (19) wenigstens teilweise Durchtritt durch diese Durchgangsöffnung (19) hindurch und am Nanowire (4) vorbei und um diesen herum bewegt werden kann, und wobei insbesondere bevorzugt eine Schaltung vorgesehen ist, welche es ermöglicht, eine Potenzialdifferenz zwischen dem Flüssigkeitsbereich auf der Oberseite und dem Flüssigkeitsbereich auf der Unterseite einzustellen.

15. Verfahren zur Messung von Eigenschaften von langkettigen Molekülen, insbesondere von DNA-Molekülen, wobei ein Sensorelement nach einem Verfahren gemäß Anspruch 14 hergestellt wird und **dadurch gekennzeichnet, dass** ein Molekül (35) auf einer Seite mit einem Widerstandselement (36) vorzugsweise in Form eines magnetischen und/oder optisch ansteuerbaren Bead, gekoppelt wird, welches eine Größe aufweist, dass es nicht durch die Durchgangsöffnung (19) hindurch treten kann, und welches durch äußere Einwirkung räumlich relativ zur Durchgangsöffnung (19) lateral zur Oberfläche verschoben werden kann, dass dieses Molekül (35) mit angekoppeltem Widerstandselement (36) in den Flüssigkeitsbereich (33) auf der Oberseite eingetragen wird, dass eine Potenzialdifferenz zwischen dem oberseitigen Flüssigkeitsbereich (33) und dem unterseitigen Flüssigkeitsbereich (34) eingestellt wird, so dass das freie Ende des Molekül (35) durch die Durchgangsöffnung (19) hindurch und in den unterseitigen Flüssigkeitsbereich (34) hindurch gezogen wird, wobei das Widerstandselement (36) im oberseitigen Flüssigkeitsbereich (33) gefangen bleibt, und dass eine äußere Einwirkung, insbesondere in Form einer Laser-single beam trap oder in Form eines magnetischen Feldes, angelegt wird, so dass das Widerstandselement (36) von der Durchgangsöffnung (19) entfernt und/oder angenähert wird, ggf. in einem alternierenden Prozess, wobei die Molekülkette (35) um den Nanowire (4) herum und an diesem kontaktierend vorbeigeführt wird, und dass eine elektrische oder elektronische Eigenschaft, vorzugsweise der sich als Funktion der Zeit ändernde Widerstand, über dem Nanowire (4) gemessen wird.

## Claims

1. A process for producing a conductive structure comprising at least one nanowire, especially a silicon nanowire (4), having a diameter of less than 50 nm, with which contacts are formed via at least two sites with electrodes (11, 13, 30), and wherein the at least one nanowire (4) and the electrodes (11, 13, 30) are arranged in one plane on a substrate (1, 29),
wherein
a) catalytically active metal nanoparticles having a diameter in the range of 0.5-50 nm are deposited on locally defined nucleation surfaces (9) on the surface (2) of an insulating substrate (1, 29),
b) the surface and the metal nanoparticles deposited thereon are exposed to a gas stream comprising at least one gaseous silicon component at a temperature in the range of 300-1100°C over a period in the range of 10-200 min, forming at least one nanowire (4) protruding from the substrate (1) and having a length in the range of 5-200 µm;
c) this at least one nanowire (4) protruding from the surface of the substrate (1) is laid down into a plane by placing a secondary substrate (5) having a contact face (6) corresponding to the surface (2) of the insulating substrate (1,29) on top;
d) and the at least one nanowire (4) laid down on the insulating substrate (1,29) contacts at two different sites with electrodes (11, 13, 30), for which a first electrode (11) is generated over the centre of the corresponding nucleation surface (9) and a second electrode (13) arranged in an annular arrangement outside the first electrode.

2. The process as claimed in claim 1, **characterized in that** the insulating substrate (1) is a substrate composed of silicon, silicon dioxide, silicon nitride or glass, and **in that** the metal nanoparticles are gold nanoparticles, preferably having a diameter in the range of 5-50 nm, preferably in the range of 20-45 nm.

3. The process as claimed in either of the preceding claims, **characterized in that**, in the course of step a), the metal nanoparticles are deposited on the substrate in spatially restricted and mutually separated regions, more preferably by applying a layer of a photoresist, especially using optical lithography, preferably producing holes having a diameter in the range of 0.02-10 µm, preferably 0.5-5 µm, in this photoresist layer via selective exposure through a structured chromium mask, and by either subsequently applying a preferably aqueous solution carrying metal nanoparticles in colloidal form, preferably nanoparticles having a diameter in the range of 0.5-500 nm, especially preferably having a diameter in the range of 5-150 nm, to the photoresist, evaporating off the solution and subsequently removing the resist with a suitable solvent, preferably acetone,
or by using electron beam metal vaporization under reduced pressure to apply a metal film, especially a gold film, to this photoresist layer, preferably having a layer thickness in the range of 0.1-2 nm, and by subsequently removing the photoresist and the metal present thereon by means of a suitable solvent, preferably acetone.

4. The process as claimed in any of the preceding claims, **characterized in that** step b) is conducted at a temperature in the range of 350-500°C, preferably in the range of 400-480°C, especially preferably in the range of 450-470°C, and by using a silane or a disilane as the gaseous silicon component, preferably in combination with a carrier gas, especially preferably nitrogen or hydrogen, preferably using a gas flow rate in the range of 50-200 sccm of silane or disilane and a gas flow rate in the range of 100-300 sccm of carrier gas, and by maintaining a total pressure in the range of 1-50 mbar, preferably in the range of 2-10 mbar, over the substrate.

5. The process as claimed in any of the preceding claims, **characterized in that**, in step a), the nanoparticles are deposited in at least one, preferably in more than one, mutually separate nucleation area (9), such that a multitude of nanowires (4) is formed in step b) over each nucleation area (9), and, in step d), a first central electrode (11) is produced over the nucleation area (9), in contact with a first end of the nanowires, preferably by means of metal vapor deposition or photolithographic deposition, and a second, second electrode (13) formed so as to at least partly surround the first electrode (11) is produced, preferably by means of metal vapor deposition or photolithographic deposition.

6. A process for producing a sensor element, especially for determination of molecular properties, **characterized in that**
a) in the substrate (21), especially in a monocrystalline silicon layer, a funnel-shaped passage orifice (19) is generated having a rectangular or square inlet orifice (28) on a top side (18) and an outlet orifice (27) on a bottom side (40) opposite the top side (18), which is smaller in terms of cross-sectional area, preferably at least five times smaller, especially preferably at least ten times smaller;
b) in a region on the top side (18) adjoining or close to the inlet orifice (28), a nanowire (4) is produced in a process as claimed in any of claims 1-5 and is placed over the inlet orifice (28) so as to form a bridge over it and contacts are formed on either side via electrodes (30);
c) the two electrodes (30) are integrated into a circuit in which an electrical or electronic property can be measured using the nanowire (4), especially the resistance as a function of time, especially as a function of the position of a molecule (35) being measured.

7. The process as claimed in claim 6, **characterized in that**, in the course of step a) of claim 6, proceeding from a silicone-based overall substrate (24) having a surface monocrystalline <100> silicon layer (21) having a thickness in the range of 5-500 nm, preferably in the range of 100-300 nm, a silicon dioxide layer (22) beneath the latter and a silicon wafer (23) beneath the latter, a funnel-shaped passage orifice in the silicon layer (21) is produced in a dry-chemical or wet-chemical etching process, wherein a square or rectangular outlet orifice (27) having a side length in the range of 2-10 nm is produced in the silicon layer (21) on the side of the silicon dioxide layer (22), by removing the silicon wafer (23) and the silicon dioxide layer (22) in the region of this outlet orifice (27) to expose the outlet orifice (27), by converting the silicon layer (21) to an insulating oxidized silicon dioxide layer (29).

8. The process as claimed in either of claims 6 and 7, **characterized in that**, in the region of the sensor element, either at the top side of the inlet orifice (28) or at the bottom side of the outlet orifice (27), a region for receiving a liquid in which molecules to be measured can be kept is provided, and wherein means, especially in the form of magnetic and/or optical and/or electrooptical and/or mechanical moving elements, are additionally provided, with which a molecule (35) which passes at least partly through the passage orifice (19) can be moved through this passage orifice (19) and past and around the nanowire (4), and wherein a circuit which enables establishment of a potential difference between the liquid region on the top side and the liquid region on the bottom side is especially preferably provided.

9. A process according to any of claims 6 - 8, wherein the substrate is a crystalline substrate and wherein in step a) of claim 6 a method for dry-chemical production of a depression (19) in the crystalline substrate (16) is used, **characterized in that** a catalyst particle (17) is deposited on a surface (18) of a crystalline substrate (16) at the site for production of the depression (19), and **in that**, in the presence of a gas atmosphere which prevents the oxidation of the substrate (16), at least the region at which the catalyst particle (17) lies on the surface (18) is exposed to a temperature of at least 500°C, preferably of at least 750°C, especially preferably of at least 900°C, most preferably in the range of 900-1100°C, over a period of at least 5 min, preferably at least 15 min, forming a funnel-shaped depression (19) which has at least three delimiting faces (20), which converge in the volume of the substrate, and which are given by crystal planes of the crystalline substrate (16).

10. The process as claimed in claim 9, **characterized in that** the substrate (16) is a monocrystalline substrate, preferably composed of silicon, preferably a (100) silicon wafer, especially an SOI structure, the delimiting faces (20) in the latter case especially preferably being four (111) crystal planes of the single crystal which run from the surface and converge in the volume of the substrate.

11. The process as claimed in either of claims 9-10, **characterized in that** the catalyst particle (17) is a metal nanoparticle, preferably a gold nanoparticle (17), preferably having a diameter in the range of 1-20 nm, especially preferably 2-10 nm, the gold nanoparticles (17) preferably being produced on the surface (18), by producing a gold layer having a thickness in the range of 0.1-2 nm on the surface, preferably using an electron beam metal vaporization process, preferably under high vacuum, and using this to form the gold nanoparticles (17) on the basis of the different surface energies.

12. The process as claimed in either of claims 6-7, **characterized in that** the depression (19) is a passage orifice through the substrate (16), by virtue of the thickness of the substrate (16) being less than the geometric depth of the depression (19) formed by the delimiting faces (20), and the depression in the surface (18) of the substrate having an inlet orifice (28) formed by the lines of intersection between the surface (18) and delimiting faces (20), and an outlet orifice (27) formed by an opposite underside surface of the substrate (16) and the delimiting faces (20), of smaller cross-sectional area, geometrically similar to the inlet orifice (28).

13. The process as claimed in claim 12, **characterized in that** the inlet orifice (28) has a polygonal, preferably rectangular or square, cross-sectional area having a side length of 50-500 nm, preferably in the range of 150-250 nm, and **in that** the depressions preferably have a geometric depth formed by the delimiting faces (20) in the range of 50-500 nm, especially preferably in the range of 150-250 nm, the geometric depth preferably being 1-50 nm, preferably 5-10 nm, greater than the thickness of the substrate, so as to form a passage orifice having an outlet orifice (27).

14. Method according to any of claims 6-13, wherein a sensor element is produced, which includes an insulating substrate (29) having a funnel-shaped passage orifice (19) having a preferably rectangular or square inlet orifice (28) on a top side (18) and a smaller outlet orifice (27) on a bottom side (40) opposite the top side (18), which is smaller in terms of cross-sectional area, preferably at least five times smaller, especially preferably at least ten times smaller, in that the at least one nanowire (4) is arranged so as to engage with and form a bridge over the inlet orifice (28) and is contact-connected on either side via electrodes (30), and these electrodes (30) are or can be integrated into a circuit in which an electrical or electronic property can be measured by means of the nanowire (4), especially the resistance as a function of time, especially as a function of the position of a molecule (35) being measured, preferably with provision of a region for receiving a liquid in which molecules to be measured can be kept either at the top side of the inlet orifice (28) or at the bottom side of the inlet orifice (27), and further preferably with additional provision of means, especially in the form of magnetic and/or optical and/or electrooptical and/or mechanical moving elements, with which a molecule (35) which passes at least partly through the passage orifice (19) can be moved through this passage orifice (19) and past and around the nanowire (4), and especially preferably with provision of a circuit which enables establishment of a potential difference between the liquid region on the top side and the liquid region on the bottom side.

15. A method for measuring properties of long-chain molecules, especially of DNA molecules, wherein a sensor element as claimed in claim 14 is made, and wherein the molecule (35) is coupled on one side to a resistance element (36), preferably in the form of a magnetic and/or optically addressable bead which is of such a size that it cannot pass through the passage orifice (19) and which can be shifted by external influence in a three-dimensional manner relative to the passage orifice (19) and laterally with respect to the surface, in that this molecule (35) coupled to the resistance element (36) is introduced into the liquid region (33) on the top side, in that a potential difference between the top-side liquid region (33) and the bottom-side liquid region (34) is established, such that the free end of the molecule (35) is pulled through the passage orifice (19) and into the bottom-side liquid region (34), the resistance element (36) remaining trapped in the top-side liquid region (33), and in that an external influence, especially in the form of a laser-single beam trap or in the form of a magnetic field, is applied, such that the resistance element (36) is moved away from and/or closer to the passage orifice (19), optionally in an alternating operation, wherein the molecular chain (35) is moved around the nanowire (4), forming contacts therewith, and in that an electrical or electronic property, preferably the change in resistance as a function of time, over the nanowire (4) is measured.

## Revendications

1. Procédé de fabrication d'une structure conductrice avec au moins un nanofil, en particulier un nanofil de silicium (4), ayant un diamètre de moins de 50 nm, qui est contacté par au moins deux points au moyen d'électrodes (11, 13, 30), et dans lequel l'au moins un nanofil (4) et les électrodes (11, 13, 30) sont disposés dans un plan sur un substrat (1, 29), dans lequel
a) on dépose sur la surface (2) d'un substrat isolant (1, 29) des nanoparticules métalliques catalytiquement actives ayant un diamètre dans la plage de 0,5 à 50 nm sur des faces de nucléation localement définies (9),
b) on expose la surface et les nanoparticules métalliques déposées sur celle-ci, à une température dans la plage de 300 à 1100°C pendant une durée dans la plage de 10 à 200 min, à un courant de gaz contenant au moins un composant de silicium gazeux, dans lequel il se forme au moins un nanofil (4) saillant sur le substrat (1) d'une longueur dans la plage de 5 à 200 pm;
c) on dépose dans un plan ledit au moins un nanofil (4) saillant sur la surface du substrat (1) par dépôt d'un substrat secondaire (5) avec une face de contact (6) correspondant à la surface (2) du substrat isolant (1, 29);
d) et on met en contact l'au moins un nanofil (4) déposé sur le substrat isolant (1, 29) en deux points différents avec des électrodes (11, 13, 30), et on produit à cet effet une première électrode (11) au-dessus du centre de la face de nucléation correspondante (9) et une deuxième électrode (13) en agencement annulaire autour de la première électrode (11) .

2. Procédé selon la revendication 1, **caractérisé en ce que** le substrat isolant (1) est un substrat en silicium, en dioxyde de silicium, en nitrure de silicium ou en verre, et **en ce que** les nanoparticules métalliques sont des nanoparticules d'or, de préférence avec un diamètre dans la plage de 5 à 50 nm, de préférence dans la plage de 20 à 45 nm.

3. Procédé selon l'une quelconque des revendications précédentes, **caractérisé en ce que** dans le cadre de l'étape a) on dépose les nanoparticules métalliques dans des zones spatialement limitées et séparées les unes des autres sur le substrat, de préférence en déposant une couche de résine photosensible, en particulier en utilisant la lithographie optique, dans lequel, de préférence par éclairage sélectif par un photomasque structuré, on produit dans cette couche de résine photosensible des trous avec un diamètre dans la plage de 0,02 à 10 µm, de préférence de 0,5 à 5 µm, et
soit en appliquant ensuite sur la résine photosensible une solution de préférence aqueuse, qui porte des nanoparticules métalliques sous forme de colloïdes, de préférence des nanoparticules avec un diamètre dans la plage de 0,5 à 500 nm, de préférence encore avec un diamètre dans la plage de 5 à 150 nm, dans lequel on évapore la solution et dans lequel on enlève ensuite la résine avec un solvant approprié, de préférence de l'acétone,
soit en appliquant sur cette couche en résine photosensible un film métallique, en particulier un film d'or, en utilisant la vaporisation du métal par faisceau d'électrons sous vide, de préférence avec une épaisseur de couche dans la plage de 0,1 à 2 nm, et en enlevant ensuite la résine photosensible et le métal se trouvant sur celle-ci au moyen d'un solvant approprié, de préférence de l'acétone.

4. Procédé selon l'une quelconque des revendications précédentes, **caractérisé en ce qu'**à l'étape b) on travaille à une température dans la plage de 350 à 500°C, de préférence dans la plage de 400 à 480°C, de préférence encore dans la plage de 450 à 470°C, et en utilisant comme composant de silicium gazeux un silane ou un disilane, de préférence en combinaison avec un gaz porteur, en particulier de préférence de l'azote ou de l'hydrogène, dans lequel on utilise de préférence un flux gazeux dans la plage de 50 à 200 sscm de silane respectivement de disilane et un flux gazeux dans la plage de 100 à 300 sscm de gaz porteur, et en maintenant une pression totale dans la plage de 1 à 50 mbar, de préférence dans la plage de 2 à 10 mbar au-dessus du substrat.

5. Procédé selon l'une quelconque des revendications précédentes, **caractérisé en ce qu'**à l'étape a) on dépose les nanoparticules dans au moins une, de préférence dans plusieurs zones de nucléation séparées les unes des autres (9), de telle manière qu'à l'étape b) il se forme une multiplicité de nanofils (4) sur chaque zone de nucléation (9), et **en ce que** dans le cadre de l'étape d) on produit une première électrode centrale (11) sur la zone de nucléation (9) en contact avec une première extrémité du nanofil, de préférence par une vaporisation de métal ou un dépôt photolithographique, et on produit une deuxième électrode (13), formée au moins en partie autour de la première électrode (11), de préférence par une vaporisation de métal ou un dépôt photolithographique.

6. Procédé de fabrication d'un élément de capteur, en particulier pour la détermination de propriétés de molécules, **caractérisé en ce que** :
a) dans le substrat (21), en particulier dans une couche de silicium monocristallin, on produit une ouverture de passage en forme d'entonnoir (19) avec une ouverture d'entrée rectangulaire ou carrée (28) sur le côté supérieur (18) et une ouverture de sortie (27), plus petite en termes de section transversale, de préférence au moins cinq fois, de préférence encore au moins dix fois plus petite, sur un côté inférieur (40) opposé au côté supérieur (18);
b) dans une région adjacente ou proche de l'ouverture d'entrée (28) sur le côté supérieur (18), on produit un nanofil (4) par un procédé selon l'une quelconque des revendications 1 à 5 et on le pose en l'occurrence au-dessus de l'ouverture d'entrée (28) pour enjamber celle-ci et on le met en contact de part et d'autre avec des électrodes (30);
c) on intègre les deux électrodes (30) dans un circuit, dans lequel on peut mesurer une propriété électrique ou électronique au moyen du nanofil (4), en particulier la résistance en fonction du temps, en particulier en fonction de la position d'une molécule mesurée (35).

7. Procédé selon la revendication 6, **caractérisé en ce que** dans le cadre de l'étape a) de la revendication 6, on produit, à partir d'un substrat total à base de silicium (24) avec une couche superficielle de silicium monocristallin <100> (21) avec une épaisseur dans la plage de 5 à 500 nm, de préférence dans la plage de 100 à 300 nm, d'une couche de dioxyde de silicium (22) disposée en dessous de celle-ci et d'une galette de silicium (23) disposée en-dessous de celle-ci, une ouverture de passage en forme d'entonnoir dans la couche de silicium (21) par un procédé de gravure chimique sèche ou humide, dans lequel on produit sur le côté de la couche de dioxyde de silicium (22) dans la couche de silicium (21) une ouverture de sortie rectangulaire ou carrée (27) avec une longueur de côté dans la plage de 2 à 10 nm, en enlevant dans la région de cette ouverture de sortie (27) la galette de silicium (23) et la couche de dioxyde de silicium (22) avec exposition de l'ouverture de sortie (27), en convertissant la couche de silicium (21) en une couche de dioxyde de silicium oxydée isolante (29).

8. Procédé selon une des revendications 6 ou 7, **caractérisé en ce qu'**il est prévu, dans la région de l'élément de capteur aussi bien sur le côté supérieur de l'ouverture d'entrée (28) que sur le côté inférieur de l'ouverture de sortie (27), une zone destinée à recevoir un liquide dans lequel des molécules à mesurer peuvent être retenues, et dans lequel il est en outre prévu des moyens, en particulier sous la forme d'éléments de mouvement magnétiques et/ou optiques et/ou électrooptiques et/ou mécaniques, avec lesquels une molécule (35), qui franchit au moins partiellement l'ouverture de passage (19), peut être déplacée à travers cette ouverture de passage (19) et le long du nanofil (4) et autour de celui-ci, et dans lequel il est de préférence encore prévu un circuit, qui permet de régler une différence de potentiel entre la zone de liquide sur le côté supérieur et la zone de liquide sur le côté inférieur.

9. Procédé selon l'une quelconque des revendications 6 à 8, dans lequel le substrat (16) est un substrat cristallin et à l'étape a) de la revendication 6, on utilise un procédé pour produire chimiquement à sec un évidement (19) dans un substrat cristallin (16), **caractérisé en ce que** l'on dépose sur une surface (18) du substrat cristallin (16) une particule de catalyseur (17) à l'endroit de l'évidement à produire (19) et **en ce que**, en présence d'une atmosphère gazeuse empêchant l'oxydation du substrat (16), on expose au moins la zone, sur laquelle la particule de catalyseur (17) est située sur la surface (18), pendant une durée d'au moins 5 min, de préférence d'au moins 15 min, à une température d'au moins 500°C, de préférence d'au moins 750°C, de préférence encore d'au moins 900°C, et de préférence encore dans la plage de 900 à 1100°C, dans lequel il se forme un évidement en forme d'entonnoir (19), dont au moins trois des faces de limitation (20) convergeant dans la profondeur du substrat sont formées par des plans cristallins du substrat cristallin (16).

10. Procédé selon la revendication 9, **caractérisé en ce que** le substrat (16) est un substrat monocristallin, de préférence en silicium, de préférence une galette de silicium (100), en particulier une structure SOI, dans lequel, de préférence encore dans ce dernier cas, les faces de limitation (20) sont quatre plans cristallins du monocristal convergeant (111) depuis la surface dans la profondeur du substrat.

11. Procédé selon une des revendications 9 et 10, **caractérisé en ce que** la particule de catalyseur (17) est une nanoparticule métallique, de préférence une nanoparticule d'or (17), de préférence avec un diamètre dans la plage de 1 à 20 nm, de préférence encore de 2 à 10 nm, dans lequel on produit de préférence la nanoparticule d'or (17) sur la surface (18) en produisant sur la surface, de préférence en utilisant un processus de vaporisation de métal par faisceau d'électrons, de préférence sous vide poussé, une couche d'or avec une épaisseur dans la plage de 0,1 à 2 nm, à partir de laquelle on forme les nanoparticules d'or (17) en raison de l'énergie de surface différente.

12. Procédé selon l'une quelconque des revendications 9 à 11, **caractérisé en ce que** l'évidement (19) est une ouverture de passage à travers le substrat (16), dans lequel l'épaisseur du substrat (16) est plus petite que la profondeur géométrique de l'évidement (19) formée par les faces de limitation (20) et dans lequel l'évidement présente dans la surface (18) du substrat une ouverture d'entrée (28) formée par les droites d'intersection entre la surface (18) et des faces de limitation (20) et une ouverture de sortie (27) formée par une surface inférieure opposée du substrat (16) et les faces de limitation (20) avec une face de section transversale plus petite géométriquement analogue à l'ouverture d'entrée (28).

13. Procédé selon la revendication 12, **caractérisé en ce que** l'ouverture d'entrée (28) présente une face de section transversale polygonale, de préférence rectangulaire ou carrée, avec une longueur de côté de 50 à 500 nm, de préférence dans la plage de 150 à 250 nm, et **en ce que** les creux présentent de préférence une profondeur géométrique, formée par les faces de limitation (20), dans la plage de 50 à 500 nm, de préférence dans la plage de 150 à 250 nm, dans lequel la profondeur géométrique est de préférence plus grande de 1 à 50 nm, de préférence de 5 à 10 nm, que l'épaisseur du substrat, de telle manière qu'une ouverture de passage avec une ouverture de sortie (27) soit formée.

14. Procédé selon l'une quelconque des revendications 6 à 13, **caractérisé en ce que** l'on fabrique un élément de capteur, qui présente un substrat isolant (29) avec une ouverture de passage en forme d'entonnoir (19) avec une ouverture d'entrée (28) de préférence rectangulaire ou carrée sur un côté supérieur (18) et une ouverture de sortie (27), plus petite en termes de face de section transversale, de préférence au moins cinq fois, de préférence encore au moins dix fois plus petite sur un côté inférieur (40) opposé au côté supérieur (18), **en ce que** l'au moins un nanofil (4) est disposé de façon accrochée à l'ouverture d'entrée (28) et enjambe celle-ci et est mis en contact de part et d'autre avec des électrodes (30), et ces électrodes (30) sont ou peuvent être intégrées dans un circuit, dans lequel une propriété électrique ou électronique peut être mesurée au moyen du nanofil (4), en particulier la résistance en fonction du temps, en particulier en fonction de la position de la molécule mesurée (35), dans lequel il est de préférence prévu aussi bien sur le côté supérieur de l'ouverture d'entrée (28) que sur le côté inférieur de l'ouverture de sortie (27) une zone destinée à recevoir un liquide, dans lequel des molécules à mesurer peuvent être retenues, et dans lequel il est en outre prévu de préférence des moyens, en particulier sous la forme d'éléments de mouvement magnétiques et/ou optiques et/ou électrooptiques et/ou mécaniques, avec lesquels une molécule (35), qui franchit au moins en partie l'ouverture de passage (19), peut être déplacée à travers cette ouverture de passage (19) et le long du nanofil (4) et autour de celui-ci, et dans lequel il est de préférence encore prévu un circuit, qui permet de régler une différence de potentiel entre la zone de liquide sur le côté supérieur et la zone de liquide sur le côté inférieur.

15. Procédé de mesure de propriétés de molécules à longue chaîne, en particulier de molécules d'ADN, dans lequel on fabrique un élément de capteur par un procédé selon la revendication 14, et **caractérisé en ce que** l'on couple une molécule (35) sur un côté avec un élément de résistance (36), de préférence sous la forme d'une perle à commande magnétique et/ou optique, qui présente une taille telle qu'elle ne peut pas passer à travers l'ouverture de passage (19), et qui peut être déplacée latéralement à la surface par une action extérieure spatialement par rapport à l'ouverture de passage (19), **en ce que** l'on introduit cette molécule (35) avec l'élément de résistance accouplé (36) dans la zone de liquide (33) sur le côté supérieur, **en ce que** l'on règle une différence de potentiel entre la zone de liquide (33) sur le côté supérieur et la zone de liquide sur le côté inférieur (34), de telle manière que l'extrémité libre de la molécule (35) soit attirée à travers l'ouverture de passage (19) et dans la zone de liquide sur le côté inférieur (34), dans lequel l'élément de résistance (36) reste prisonnier dans la zone de liquide sur le côté supérieur (33), et **en ce que** l'on applique une action extérieure, en particulier sous la forme d'un piège à faisceau laser unique ou sous la forme d'un champ magnétique, de telle manière que l'élément de résistance (36) soit éloigné et/ou approché de l'ouverture de passage (19), éventuellement dans un processus alternatif, dans lequel la chaîne moléculaire (35) est menée autour du nanofil (4) et en contact le long de celui-ci, et **en ce que** l'on mesure une propriété électrique ou électronique, de préférence la résistance variable en fonction du temps, au moyen du nanofil (4).
